(19) Europäisches Patentamt
European Patent Office
Office européen des brevets

(11) Veröffentlichungsnummer: **0 253 186 B1**

# (12) EUROPÄISCHE PATENTSCHRIFT

(45) Veröffentlichungstag der Patentschrift: **19.06.91**

(51) Int. Cl.5: **C07C 275/64**, D06M 13/425

(21) Anmeldenummer: **87109287.0**

(22) Anmeldetag: **27.06.87**

## (54) Urethane aus aliphatischen Fluoralkoholen, Isocyanaten und aromatischen Verbindungen, Verfahren zu ihrer Herstellung und ihre Verwendung.

(30) Priorität: **03.07.86 DE 3622284**

(43) Veröffentlichungstag der Anmeldung:
**20.01.88 Patentblatt 88/03**

(45) Bekanntmachung des Hinweises auf die Patenterteilung:
**19.06.91 Patentblatt 91/25**

(84) Benannte Vertragsstaaten:
**AT BE CH DE ES FR GB IT LI NL**

(56) Entgegenhaltungen:
**EP-A- 0 172 717**

(73) Patentinhaber: **HOECHST AKTIENGESELLSCHAFT**
**Postfach 80 03 20**
**W-6230 Frankfurt am Main 80(DE)**

(72) Erfinder: **Wehowsky, Frank, Dr.**
**Talhauser Strasse 27**
**W-8269 Burgkirchen(DE)**
Erfinder: **Kleber, Rolf, Dr.**
**Am Trieb 41**
**W-6078 Neu-Isenburg(DE)**
Erfinder: **Jaeckel, Lothar**
**Ketteler Strasse 88**
**W-6093 Flörsheim am Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft Urethane, die aus aliphatischen Fluoralkoholen, Isocyanaten und aromatischen Verbindungen zusammengesetzt sind. Die Erfindung betrifft ferner ein Verfahren zur Herstellung dieser Urethane und ihre Verwendung.

Aus der europäischen Patentanmeldung 0 172 717-A2 ist es bekannt, Urethane aus mindestens einem aliphatischen Fluoralkohol mit einer Perfluoralkylgruppe als Fluorkomponente und einem Tris-(isocyanatoalkan)biuret als Isocyanatkomponente durch Inkorporierung einer modifizierenden Gruppe in den Eigenschaften bezüglich Textilausrüstung zu verbessern. Die modifizierende Gruppe kann eine aromatische, aliphatische, alicyclische Verbindung oder eine Mischung von solchen Verbindungen mit einem oder mehreren aktiven Wasserstoffatomen sein (über die aktiven Wasserstoffatome erfolgt die Verknüpfung der Verbindungen mit Isocyanatgruppen). Von den genannten Modifiern werden im wesentlichen nur aliphatische Verbindungen näher beschrieben.

Es wurde nun überraschenderweise gefunden, daß Perfluoralkylgruppen und gegebenenfalls Epichlorhydringruppen enthaltende Urethane dann besonders gute Eigenschaften im Hinblick auf die Ausrüstung von Textilien besitzen, wenn sie zusätzlich auch noch solche aromatische Gruppen enthalten, die von aktive Wasserstoffatome enthaltenden aromatischen Verbindungen, ausgewählt aus der Gruppe der aromatischen Dihydroxy-, Diamino-, Aminohydroxy-, Aminocarboxy- und Hydroxycarboxy-Verbindungen, stammen. Die neuen Urethan-Verbindungen enthalten also im Molekül mindestens eine Perfluoralkylgruppe und mindestens eine durch ein aktives Wasserstoffatom addierte spezielle aromatische Verbindung der genannten Art sowie gegebenenfalls mindestens eine Epichlorhydringruppe.

Die erfindungsgemäßen Urethan-Verbindungen entsprechen der nachstehenden allgemeinen Formel 1

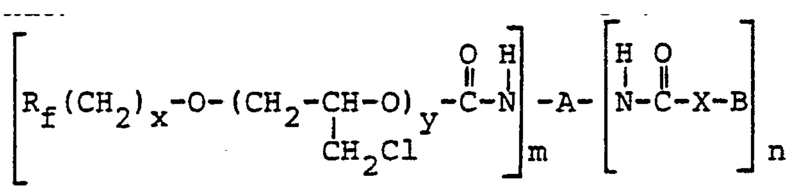 (1)

worin bedeuten:

- $R_f$ eine Perfluoralkylgruppe mit 4 bis 20 C-Atomen, vorzugsweise 6 bis 16 C-Atomen, oder eine $R'_fSO_2NR_1$-Gruppe, in der $R'_f$ eine der Bedeutungen von $R_f$ hat und $R_1$ H oder eine Alkylgruppe mit 1 bis 4 C-Atomen ist,
- x eine ganze Zahl von 1 bis 4, vorzugsweise 2,
- y eine Zahl von 0 bis 10, vorzugsweise 1 bis 5,
- m eine Zahl von 1 bis 2 und
- n eine Zahl von 1 bis 2, wobei die Summe aus m + n höchstens 3 ist,
- A eine der Gruppen entsprechend den nachstehenden Formeln 2 bis 10 (das sind isocyanatfreie Reste):

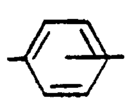 (2) 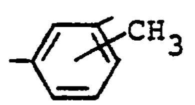 (3)

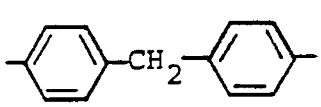 (4) 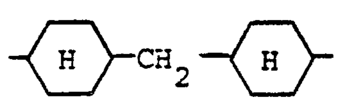 (5)

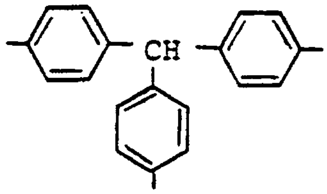 (6)

$-(CH_2)_6-$ (7)

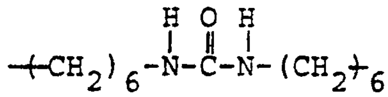 (8)

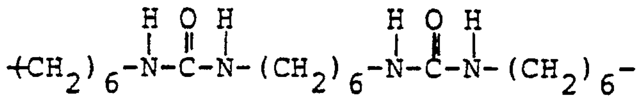 (9)

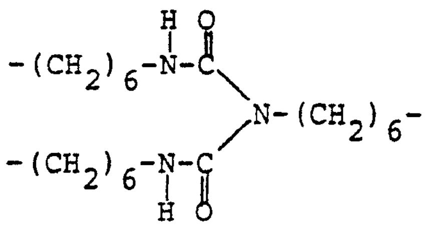 (10)

X eine der Gruppen entsprechend den nachstehenden Formeln 11 bis 19, die mit einer Alkylgruppe mit 1 bis 4 C-Atomen ein- oder mehrfach, vorzugsweise einfach, substituiert sein können (die Formeln 11 bis 19 repräsentieren Reste von gegebenenfalls mit $C_1$- bis $C_4$-Alkylgruppen substituierten aromatischen Dihydroxy-, Diamino-, Aminohydroxy-, Aminocarboxy- und Hydroxycarboxy-Verbindungen, die nach Abgabe von aktiven Wasserstoffatomen an Isocyanatgruppen vorliegen):

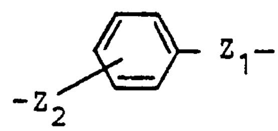 (11)

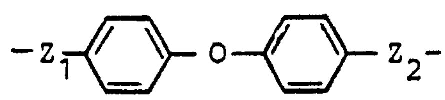 (12)

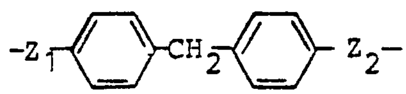 (13)

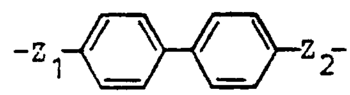 (14)

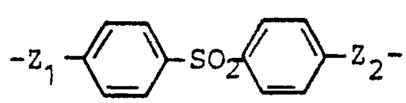 (15)

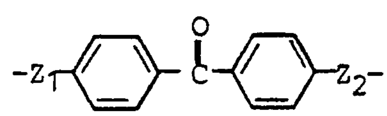 (16)

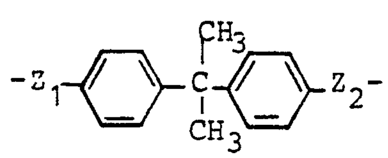 (17)

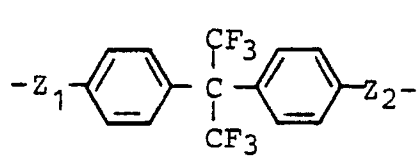 (18)

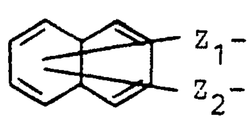 (19) ,

worin $Z_1$ und $Z_2$ für O, NH oder COO stehen, wobei die beiden Substituenten nicht gleich sind, oder für O oder NH stehen, wobei die beiden Substituenten auch gleich sein können,
und

B das Wasserstoffatom, eine Alkylgruppe mit 1 bis 4 C-Atomen oder eine Gruppe entsprechend der nachstehenden Formel 20

$$\left[R''_f-(CH_2)_{x'}-O-(CH_2-\underset{\displaystyle CH_2Cl}{\underset{|}{CH}}-O)_{y'}-\overset{O}{\overset{\|}{C}}-\overset{H}{\overset{|}{N}}\right]_{m'}A'\left(\overset{H}{\overset{|}{N}}-\overset{O}{\overset{\|}{C}}\right)_{n'} \quad (20),$$

worin $R''_f$, x', y', m', n' und A' eine der Bedeutungen von $R_f$, x, y, m, n und A haben.

Von den angegebenen Bedeutungen für $R_f$ ist die Perfluoralkylgruppe mit 4 bis 20 C-Atomen, vorzugsweise 6 bis 16 C-Atomen, bevorzugt. Die Perfluoralkylgruppe kann geradkettig oder verzweigt sein; im Falle einer verzweigten Perfluoralkylgruppe ist die endverzweigte bevorzugt. Von den beiden Perfluoralkylgruppen, den geradkettigen oder verzweigten, sind die geradkettigen bevorzugt. Beim Perfluoralkyl-Rest handelt es sich in der Regel um ein Gemisch von Perfluoralkylgruppen mit der obengenannten Anzahl von C-Atomen.

A ist vorzugsweise eine Toluylengruppe oder eine der drei Gruppen entsprechend den Formeln 8 bis 10 (diese drei Gruppen liegen in der Regel als Gemisch vor). X ist vorzugsweise eine Gruppe entsprechend den Formeln 11, 15, 16, 17 oder 18.

B ist vorzugsweise Wasserstoff oder eine Gruppe entsprechend der Formel 20, wobei A' eine der drei Gruppen entsprechend den Formeln 8 bis 10 ist.

Die Herstellung der erfindungsgemäßen Urethane ergibt sich aus der allgemeinen Formel 1 und wird im folgenden näher beschrieben. Sie werden hergestellt durch Reaktion eines aliphatischen Fluoralkohols der Formel

$$R_f(CH_2)_xO(CH_2\underset{\displaystyle CH_2Cl}{\underset{|}{CHO}})_y-H \quad ,$$

worin $R_f$, x und y die obengenannte Bedeutung haben,
mit einem Di- oder Triisocyanat entsprechend einer der Gruppen der Formeln 2 bis 10 zum Addukt der Formel

$$\left[R_f(CH_2)_xO(CH_2\underset{\displaystyle CH_2Cl}{\underset{|}{CHO}})_y-CONH\right]_m A-(NCO)_n \quad (\text{Addukt } 1),$$

worin $R_f$, x, y, m, n und A die obengenannte Bedeutung haben,
und durch Reaktion des Adduktes 1 mit einer aromatischen Dihydroxy-, Diamino-, Aminohydroxy-, Aminocarboxy- oder Hydroxycarboxy-Verbindung der nachstehenden Formeln

H-X-H oder H-X-B ,

worin X eine der Gruppen der Formeln 11 bis 19 und B eine Alkylgruppe mit 1 bis 4 C-Atomen ist,
zu den erfindungsgemäßen Urethanen der beiden nachstehenden Formeln 21 und 22

$$\left[R_f(CH_2)_xO(\underset{\displaystyle CH_2Cl}{CH_2CHO})_y-CONH\right]_m-A-\left[NHCO-X-H\right]_n \qquad (21)$$

$$\left[R_f(CH_2)_xO(\underset{\displaystyle CH_2Cl}{CH_2CHO})_y-CONH\right]_m-A-\left[NHCO-X-B\right]_n \qquad (22),$$

worin $R_f$, x, y, m, n und A die obengenannte Bedeutung haben und B die genannte Alkylgruppe mit 1 bis 4 C-Atomen ist,
und durch Reaktion eines Urethans der Formel 21 mit einer eine oder mehrere freie Isocyanatgruppen enthaltenden Isocyanat-Verbindung entsprechend der Gruppe der Formel 20 zu den angestrebten erfindungsgemäßen Urethanen der nachstehenden Formel 23

$$\left[R_f(CH_2)_xO(\underset{\displaystyle CH_2Cl}{CH_2CHO})_y-CONH\right]_m-A-\left\{NHCO-X-(CONH)_{n'}A'-\left[NHCO-(O\underset{\displaystyle CH_2Cl}{CHCH_2})_{y'}-O(CH_2)_{x'}-R_f''\right]_{m'}\right\}_n \qquad (23),$$

worin $R_f$, $R_f''$, x, x', y, y', m, m', n, n', A, A' und X die obengenannte Bedeutung haben.

Im folgenden wird die Herstellung der erfindungsgemäßen Verbindungen im einzelnen beschrieben.

Zur Herstellung des Adduktes 1 werden aliphatische Fluoralkohole mit einer Perfluoralkyl-Gruppe in Form eines Perfluorhydroalkanols oder Perfluorsulfonamidoalkanols und gegebenenfalls mit mindestens einer Epichlorhydrin-Gruppe (entsprechend der Bedeutung von y in Formel 1) eingesetzt. Perfluorhydroalkanole und Perfluorsulfonamidoalkanole, wie sie zur Herstellung des Adduktes 1 eingesetzt werden, wenn in Formel 1 y = Null ist, sind schon seit langem bekannt und brauchen deshalb nicht mehr näher beschrieben zu werden. Die aliphatischen Fluoralkohole mit einer Perfluoralkyl-Gruppe und mit Epichlorhydrin-Gruppen werden dadurch erhalten, daß man beispielsweise Perfluoralkylethanol (als Perfluorhydroalkanol) oder beispielsweise Perfluoralkylsulfonamidoethanol (als Perfluorsulfonamidoalkanol) mit Epichlorhydrin (Siedepunkt bei Normalbedingungen 116 °C), gegebenenfalls in Gegenwart von Lewis-Säuren als Katalysator,bei einer Temperatur von 30 bis 100 °C, vorzugsweise 40 bis 70 °C, umsetzt, wobei die Ethanolverbindung und das Epichlorhydrin im Molverhältnis von etwa 1 : y eingesetzt werden (y hat die in Rede stehende Bedeutung). Bei Perfluorhydroalkanol und beim Perfluorsulfonamidoalkanol handelt es sich bezüglich Perfluoralkylrest in der Regel um preisgünstige, im Handel erhältliche Gemische mit im wesentlichen 6 bis 20 C-Atomen. Die Art der Lewis-Säure ist nicht kritisch. Bevorzugt sind $BF_3$, Bortrifluoriddiethyletherat, $SnCl_4$, $SbCl_5$, $TiCl_4$, $FeCl_3$, $PF_5$ und/oder Dibutylzinndilaurat, wobei Bortrifluoriddiethyletherat besonders bevorzugt ist. Die Menge an Katalysator beträgt im allgemeinen 0,01 bis 5 Gew.-%, vorzugsweise 0,1 bis 1 Gew.-%, bezogen auf Perfluoralkylethanol. Die Umsetzung wird vorzugsweise unter Rühren und bei dem sich einstellenden Druck durchgeführt. Die Umsetzungsdauer liegt im Bereich von etwa 0,5 bis 7 h. Es kann zweckmäßig sein, ein Lösungsmittel einzusetzen. Bevorzugte Lösungsmittel sind halogenierte Kohlenwasserstoffe wie Tetrachlorkohlenstoff, Trichlorethylen, 1,2-Dichlorethan, Trichlorethan und Trifluortrichlorethan; Ketone wie Methylethylketon und Cyclohexanon; Ether wie Diisopropylether und Tetrahydrofuran. Die in Rede stehende Umsetzung läuft quantitativ ab. Im erhaltenen Reaktionsprodukt wird das gegebenenfalls verwendete Lösungsmittel abdestilliert, wobei auch gegebenenfalls vorhandene flüchtige Anteile wie nichtumgesetztes Epichlorhydrin entfernt werden. Aus Zweckmäßigkeitsgründen kann man die Destillation auch unter Vakuum (Wasserstrahl-Vakuum) durchführen. Die als Katalysator eingesetzte Lewis-Säure, die bei der nachfolgenden Umsetzung mit Isocyanat an sich nicht stört, kann mit Hilfe von alkalischen Mitteln,

vorzugsweise mit Hilfe einer wäßrigen Natriumbicarbonat-Lösung oder einem Amin wie Triethylamin, ausgewaschen bzw. neutralisiert werden. Der aliphatische Fluoralkohol mit einer Perfluoralkyl-Gruppe und mit Epichlorhydrin-Gruppen stellt ein wachsartiges, gelbgefärbtes Produkt dar.

Zur Herstellung des Adduktes 1 wird vorzugsweise so vorgegangen, daß man einen aliphatischen Fluoralkohol mit einer Perfluoralkyl-Gruppe und gegebenenfalls mit mindestens einer Epichlorhydrin-Gruppe (entsprechend der Bedeutung von y in Formel 1) mit einem Isocyanat entsprechend den Formeln 2 bis 10 bei einer Temperatur von 70 bis 150 °C, vorzugsweise 90 bis 130 °C, umsetzt, wobei der aliphatische Fluoralkohol und das Isocyanat in dem Molverhältnis eingesetzt werden, das sich aus der angestrebten Bedeutung für m und n in der Formel für das Addukt 1 ergibt. Die Umsetzung wird vorzugsweise unter Rühren und bei dem sich einstellenden Druck durchgeführt und - sofern es zweckmäßig ist, beispielsweise zur Verkürzung der Reaktionszeit - in Gegenwart der obengenannten Lewis-Säure-Katalysatoren. Es können auch Lösungsmittel, z. B. Ester, eingesetzt werden. Die Umsetzungsdauer liegt im Bereich von 1 bis 15 h. Bei dem Isocyanat wird es sich oft um im Handel erhältliche Isocyanat-Gemische handeln. So besteht das Toluylendiisocyanat in der Regel aus etwa 80 Gew.-% 2,4-Toluylendiisocyanat und 20 Gew.-% 2,6-Toluylendiisocyanat. Auch die Isocyanate entsprechend den Gruppen der Formeln 8 bis 10 liegen in der Regel als Gemisch vor. Ein im Handel erhältliches und bevorzugtes derartiges Gemisch besteht aus den drei in Rede stehenden Isocyanaten, wobei das Isocyanat entsprechend der Formel 10 in einer Menge von mindestens 50 Gew.-%, bezogen auf die Mischung, vorhanden ist (in dieser Mischung ist also das Isocyanat der Formel 10 die Hauptkomponente). Die Umsetzung des in Rede stehenden aliphatischen Fluoralkohols mit Isocyanat zum Addukt 1 verläuft quantitativ. Die erhaltenen Produkte können gegebenenfalls gereinigt werden, beispielsweise können flüchtige Anteile abdestilliert werden. Das Addukt 1 stellt ein wachsartiges, gelbgefärbtes Produkt dar.

Zur Herstellung der erfindungsgemäßen Verbindungen der Formeln 21 und 22 wird vorzugsweise so vorgegangen, daß man das Addukt 1 mit einer aromatischen aktive Wasserstoffatome enthaltenden Verbindung der oben angegebenen Formeln H-X-H oder H-X-B bei einer Temperatur von 70 bis 150 °C, vorzugsweise 90 bis 130 °C, umsetzt, wobei die Addukt-1-Verbindung und die aktive Wasserstoffatome tragende aromatische Verbindung in einem solchen Molverhältnis eingesetzt werden, daß die Molmenge an aromatischer Verbindung den in dem eingesetzten Addukt 1 vorhandenen freien Isocyanat-Gruppen entspricht. Die Umsetzung wird vorzugsweise unter Rühren und bei dem sich einstellenden Druck durchgeführt und - sofern es zweckmäßig ist, beispielsweise zu Verkürzung der Reaktionszeit - in Gegenwart der obengenannten Lewis-Säure-Katalysatoren. Es können auch Lösungsmittel, z. B. Ester, eingesetzt werden. Die Umsetzungsdauer liegt im Bereich von 1 bis 40 h. Die Umsetzung des Adduktes 1 mit der aktive Wasserstoffatome tragenden aromatischen Verbindung zu den erfindungsgemäßen Verbindungen der Formeln 21 und 22 verläuft quantitativ. Das erhaltene Produkt kann gegebenenfalls gereinigt werden, beispielsweise können flüchtige Anteile abdestilliert werden. Die erfindungsgemäßen Verbindungen nach den Formeln 21 und 22 stellen wachsartige, gelb- bis braungefärbte Produkte dar.

Zur Herstellung der erfindungsgemäßen Verbindungen der Formel 23 wird vorzugsweise so vorgegangen, daß man eine Verbindung der Formel 21 mit einer Isocyanat-Verbindung entsprechend der Gruppe gemäß Formel 20 (vergleiche Addukt 1) bei einer Temperatur von 70 bis 150 °C, vorzugsweise 90 bis 130 °C, umsetzt, wobei die Verbindung nach Formel 21 und die Isocyanat-Verbindung in einem solchen Molverhältnis eingesetzt werden, daß die Molmenge an Isocyanat-Verbindung den in der eingesetzten Verbindung der Formel 21 vorhandenen aktiven Wasserstoffatomen entspricht. Im übrigen gilt auch für die Herstellung dieser erfindungsgemäßen Verbindungen das,was oben über die Herstellung der erfindungsgemäßen Verbindungen nach den Formeln 21 und 22 gesagt worden ist. Die erfindungsgemäßen Verbindungen nach Formel 23 stellen, ebenso wie jene nach den Formeln 21 und 22, wachsartige, gelb- bis braungefärbte Produkte dar.

Die erfindungsgemäßen Verbindungen stellen überraschend gute Textilbehandlungsmittel dar. Sie verleihen den Textilien vor allem eine hervorragende Hydrophobie und Oleophobie. Sie weisen ferner in einem hohen Ausmaß die Eigenschaft auf, den starken Beanspruchungen, denen die ausgerüsteten Textilien ausgesetzt werden, beispielsweise beim Verstrecken, Texturieren und insbesondere beim Färben und Waschen, ohne irgendeinen Verlust an Wirkung standzuhalten. Ein unerwarteter und besonders großer Vorteil der erfindungsgemäßen Verbindungen liegt darin, daß sie auch in üblichen Textilbehandlungspräparationen, beispielsweise in Spinnpräparationen, eingesetzt werden können und dabei ihre hervorragende Wirkung nicht verlieren.

Das Textilmaterial kann natürlicher und/oder synthetischer Natur sein. Es besteht vorzugsweise aus Polyamid, Polyester und/oder Polyacrylnitril, wobei Polyamid besonders bevorzugt ist. Das Textilmaterial kann in beliebiger Form vorliegen, so zum Beispiel als Faden, Faser, Garn, Flocke, Gewebe, Gestrick, Gewirk, Teppich oder Vlies. Die Auftragsmenge an erfindungsgemäßer Verbindung wird so gewählt, daß auf

dem Textilmaterial 0,02 bis 1 Gew.-% Fluor, vorzugsweise 0,04 bis 0,4 Gew.-% Fluor, vorhanden sind, berechnet aus der Menge an Fluor in der erfindungsgemäßen Verbindung; Gewichtsprozente bezogen auf das behandelte Textilmaterial. Die Behandlung des Textilmaterials mit den erfindungsgemäßen Urethanen erfolgt in der Regel entweder über die oben erwähnten Textilbehandlungspräparationen, denen die erfindungsgemäßen Urethane einverleibt worden sind, oder mit Hilfe von Lösungen, Emulsionen oder Dispersionen, die aus den Urethanen eigens bereitet worden sind. In den Lösungen, Emulsionen oder Dispersionen bzw. in den Textilbehandlungspräparationen liegen sie in der Regel in einer Konzentration von 5 bis 40 bzw. 0,5 bis 5 Gew.-% vor, vorzugsweise 8 bis 30 bzw. 1 bis 3 Gew.-%.

Die Behandlung der Textilien mit den genannten Lösungen, Emulsionen oder Dispersionen wird nach üblichen Methoden durchgeführt, so zum Beispiel durch Sprühen, Tauchen, Foulardieren und dergleichen. Anschließend wird das getränkte Textilmaterial getrocknet und einer Wärmebehandlung unterworfen. Die Wärmebehandlung wird in der Regel in der Weise durchgeführt, daß das Textilmaterial auf eine Temperatur von 130 bis 200 °C erhitzt und bei dieser Temperatur 10 Sekunden bis 10 Minuten lang gehalten wird. Das mit den erfindungsgemäßen Urethanen ausgerüstete Textilmaterial besitzt die oben erwähnten hervorragenden Eigenschaften.

Die erfindungsgemäßen Verbindungen sind auch zur hydrophoben und oleophoben Ausrüstung von Leder hervorragend geeignet. Als Beispiele für Leder seien Rind-, Ziegen-, Schafund Schweineleder genannt. Die Auftragsmenge an erfindungsgemäßer Verbindung wird so gewählt, daß auf dem Leder 0,05 bis 1,5 Gew.-% Fluor, vorzugsweise 0,1 bis 1 Gew.-% Fluor, vorhanden sind, berechnet aus der Menge an Fluor in der Verbindung; Gewichtsprozente bezogen auf das behandelte Leder. Zum Aufbringen können die üblichen Arbeitsweisen zur Ausrüstung von Leder eingesetzt werden.

Die Erfindung wird nun anhand von Beispielen noch näher erläutert.

Erfindungsgemäße Verbindungen

Beispiel 1

In einem mit einem Rührer, Rückflußkühler, Thermometer, Tropftrichter und Heizbad ausgestatteten Glaskolben wurden 8 kg (15,7 mol) von einem im Handel erhältlichen Perfluoralkylethanol-Gemisch mit Perfluoralkyl = $C_8F_{17}$-$C_{16}F_{33}$ (OH-Zahl = 106), 8 kg 1,2,2-Trifluortrichlorethan ($CFCl_2$-$CF_2Cl$; Kp = 48 °C) als Lösungsmittel und 50 g Bortrifluoriddiethyletherat als Katalysator (das sind 0,6 Gew.-% Katalysator, bezogen auf Perfluoralkylethanol) vorgelegt. Zu dieser Lösung wurden bei 45 °C 2,9 kg (31,4 mol) Epichlorhydrin zugetropft, worauf 3 Stunden lang bei der Siedetemperatur des Lösungsmittels gehalten wurde. Anschließend wurde das eingesetzte Lösungsmittel im Vakuum (Wasserstrahlvakuum) abdestilliert. Es lag ein wachsartiges gelb gefärbtes Produkt vor. In dem so erhaltenen aliphatischen Fluoralkohol ist das Molverhältnis von Perfluoralkylethanol zu Epichlorhydrin = 1 : 2 (y in Formel 1 hat den Wert 2, der sich durch Mittelwertbildung von 1 bis 8 angelagerten Epichlorhydrin-Einheiten ergibt).

Die Umsetzung des aliphatischen Fluoralkohols mit Isocyanat zum Fluoralkohol-Isocyanat-Addukt (Addukt 1) erfolgte in einem mit Rührer, Rückflußkühler mit Trockenrohr, Thermometer und Heizbad ausgestatteten Glaskolben. Es wurden 85,0 g (0,13 val) vom aliphatischen Fluoralkohol und 35,7 g (0,19 val) Triisocyanat entsprechend Formel 10, und zwar ein als Handelsprodukt erhältliches Gemisch aus den drei Isocyanaten entsprechend den Formeln 8, 9 und 10 mit dem Triisocyanat als Hauptbestandteil, vorgelegt (das ist ein Molverhältnis von 2 : 1) und 4 Stunden lang bei 110 °C unter Rühren gehalten. Zur Mischung wurden nun 5 Tropfen Dibutylzinndilaurat dazugegeben, worauf sie zur Nachreaktion 3 Stunden lang bei 110 °C unter Rühren gehalten wurde. Das erhaltene Fluoralkohol-Isocyanat-Addukt (Addukt 1) war ein wachsartiges, gelb gefärbtes Produkt.

Die Umsetzung des Adduktes 1 mit der aromatischen difunktionellen Verbindung erfolgte ebenfalls in einem mit Rührer, Rückflußkühler mit Trockenrohr, Thermometer und Heizbad ausgestatteten Glaskolben. Es wurden 100,0 g (62,6 mval) vom Addukt 1 und 3,5 g (62,8 mval) Brenzcatechin vorgelegt (das ist ein Molverhältnis von 2 : 1) und 35 Stunden lang bei 110 °C unter Rühren gehalten. Es wurden 100,6 g, das sind 97,3 Gew.-% der Theorie,erfindungsgemäße Verbindung in Form eines wachsartigen, braun gefärbten Produktes erhalten. Die Bruttozusammensetzung der erfindungsgemäßen Verbindung mit einem aliphatischen Fluoralkohol, Isocyanat und Brenzcatechin im Molekül (Molverhältnis 4 : 2 : 1) entspricht der in der Tabelle nach den Beispielen angegebenen Formel B1.

Beispiele 2 und 3

Es wurde wie in Beispiel 1 vorgegangen, mit der Ausnahme, daß anstelle von Brenzcatechin Resorcin

(Beispiel 2) und Hydrochinon (Beispiel 3) eingesetzt wurden, und daß nicht 35 Stunden, sondern jeweils 10 Stunden bei 110 °C gehalten wurde. Es wurden jeweils 102,0 g, das sind 99,0 Gew.-% der Theorie, erfindungsgemäße Verbindung in Form eines wachsartigen, braun gefärbten Produktes erhalten. Die Bruttozusammensetzung der beiden erfindungsgemäßen Verbindungen mit einem aliphatischen Fluoralkohol, Isocyanat und Resorcin beziehungsweise Hydrochinon im Molekül (Molverhältnis jeweils 4 : 2 : 1) entsprechen den in der genannten Tabelle angegebenen Formeln B2 und B3.

Beispiel 4

Es wurden 231,4 g (0,12 val) vom Addukt 1 des Beispiels 1 und 14,2 g (0,12 val) 2,2-(4,4'-Dihydroxydiphenyl)-propan, das ist Bisphenol A (das ist ein Molverhältnis von 2 : 1) und 62,0 g Adipinsäure-di-n-butylester als Lösungsmittel in den oben angegebenen Glaskolben vorgelegt und 12 Stunden lang bei 110 °C unter Rühren gehalten. Zur Mischung wurden nun 5 Tropfen Dibutylzinndilaurat dazugegeben, worauf sie zur Nachreaktion 4 Stunden lange bei 110 °C unter Rühren gehalten wurde. Es wurden 304,0 g, das sind 99,0 Gew.-% der Theorie, erfindungsgemäße Verbindung in Form eines wachsartigen, braun gefärbten Produktes erhalten. Die Bruttozusammensetzung der erfindungsgemäßen Verbindung mit einem aliphatischen Fluoralkohol, Isocyanat und Bisphenol A im Molekül (Molverhältnis 4 : 2 : 1) entspricht der in der genannten Tabelle angegebenen Formel B4.

Beispiel 5

Ansatz:
154,3 g (80 mval) vom Addukt 1 des Beispiels 1
13,4 g (80 mval) 2,2-(4,4'-Dihydroxydiphenyl)-hexafluorpropan (Hexafluorbisphenol A)
42,0 g Adipinsäure-di-n-butylester.
Durchführung wie in Beispiel 4.
Ausbeute: 205,8 g, das sind 98,1 Gew.-% der Theorie, an erfindungsgemäßer Verbindung in Form eines wachsartigen, braun gefärbten Produktes. Die Bruttozusammensetzung der erfindungsgemäßen Verbindung mit einem aliphatischen Fluoralkohol, Isocyanat und Hexafluorbisphenol A im Molekül (Molverhältnis 4 : 2 : 1) entspricht der Formel B5.

Beispiel 6

Ansatz:
238,5 g (124,0 mval) vom Addukt 1 des Beispiels 1
15,3 g (124,0 mval) Bis(4-hydroxyphenyl)sulfon
64,0 g Adipinsäure-di-n-butylester.
Durchführung wie in Beispiel 4.
Ausbeute: 314,0 g, das sind 98,5 Gew.-% der Theorie, an erfindungsgemäßer Verbindung in Form eines wachsartigen, braun gefärbten Produktes. Die Bruttozusammensetzung der erfindungsgemäßen Verbindung mit einem aliphatischen Fluoralkohol, Isocyanat und Bis(4-hydroxyphenyl)sulfon im Molekül (Molverhältnis 4 : 2 : 1) entspricht der Formel B6.

Beispiel 7

Ansatz:
100,0 g (63,0 mval) vom Addukt 1 des Beispiels 1
3,4 g (63,0 mval) 3-Aminophenol.
Durchführung wie in Beispiel 1 mit der Ausnahme, daß nicht 35 Stunden sondern 6 Stunden bei 110 °C gehalten wurde.
Ausbeute: 102,0 g, das sind 98,7 Gew.-% der Theorie, an erfindungsgemäßer Verbindung in Form eines wachsartigen, braun gefärbten Produktes. Die Bruttozusammensetzung der erfindungsgemäßen Verbindung mit einem aliphatischen Fluoralkohol, Isocyanat und 3-Aminophenol im Molekül (Molverhältnis 4 : 2 : 1) entspricht der Formel B7.

Beispiel 8

Zur Herstellung eines weiteren Adduktes 1 wurden in dem in Beispiel 1 angegebenen Glaskolben 162,0

g (0,32 val) vom Perfluoralkylethanol des Beispiels 1, 216,0 g (0,32 val) vom aliphatischen Fluoralkohol des Beispiels 1 und 181,3 g (0,96 val) des Triisocyanats vom Beispiel 1 vorgelegt (das ist ein Molverhältnis von 1 : 1 : 1) und 5 Stunden lang bei 110 °C unter Rühren gehalten. Das erhaltene Fluoralkohol-Isocyanat-Addukt (Addukt 1) war ein wachsartiges, gelb gefärbtes Produkt.

Zur Umsetzung dieses Adduktes 1 mit einer aromatischen difunktionellen Verbindung wurden analog Beispiel 1 559,0 g (0,32 val) von dem Addukt 1, 17,5 g (0,32 val) Hydrochinon und 144,0 g Adipinsäure-di-n-butylester als Lösungsmittel vorgelegt (das ist ein Molverhältnis von 2 : 1) und 10 Stunden lang bei 110 °C unter Rühren gehalten. Es wurden 715,0 g, das sind 99,2 Gew.-% der Theorie, erfindungsgemäße Verbindung in Form eines wachsartigen, braun gefärbten Produktes erhalten. Die Bruttozusammensetzung der erfindungsgemäßen Verbindung mit einem aliphatischen Fluoralkohol, Isocyanat und Hydrochinon im Molekül (Molverhältnis 4 : 2 : 1),entspricht der Formel B8.

Beispiel 9

Ansatz:
289,3 g (0,15 val) vom Addukt 1 des Beispiels 1
37,5 g (0,3 val) Bisphenol A.
Durchführung wie in Beispiel 1 mit der Ausnahme, daß nicht 35 Stunden, sondern 7 Stunden bei 110 °C gehalten wurde, und daß nach Zugabe von 5 Tropfen Dibutylzinndilaurat zur Nachreaktion weitere 6 Stunden lang bei 110 °C unter Rühren gehalten wurde.
Ausbeute: 318,0 g, das sind 97,5 Gew.-% der Theorie, an erfindungsgemäßer Verbindung in Form eines wachsartigen, braun gefärbten Produktes. Die Bruttozusammensetzung der erfindungsgemäßen Verbindung mit einem aliphatischen Fluoralkohol, Isocyanat und Bisphenol A im Molekül (Molverhältnis 2 : 1 : 1) entspricht der Formel B9.

Beispiel 10

Ansatz:
289,3 g (0,15 val) vom Addukt 1 des Beispiels 1
37,5 g (0,3 val) Bis(4-hydroxyphenyl)sulfon.
Durchführung wie in Beispiel 9.
Ausbeute: 323,4 g, das sind 99,0 Gew.-% der Theorie, an erfindungsgemäßer Verbindung in Form eines wachsartigen, braun gefärbten Produktes. Die Brutttozusammensetzung der erfindungsgemäßen Verbindung mit einem aliphatischen Fluoralkohol, Isocyanat und Bis(4-hydroxyphenyl)sulfon im Molekül (Molverhältnis 2 : 1 : 1) entspricht der Formel B10.

Beispiel 11

Ansatz:
529,0 g (0,3 val) vom Addukt 1 des Beispiels 8
33,0 g (0,6 val) Hydrochinon.
Durchführung wie in Beispiel 8.
Ausbeute: 553,0 g, das sind 98,5 Gew.-% der Theorie, an erfindungsgemäßer Verbindung in Form eines wachsartigen, braun gefärbten Produktes. Die Bruttozusammensetzung der erfindungsgemäßen Verbindung mit einem aliphatischen Fluoralkohol, Isocyanat und Hydrochinon im Molekül (Molverhältnis 2 : 1 : 1) entspricht der Formel B11.

Beispiel 12

Ansatz:
289,3 g (0,15 val) vom Addukt 1 des Beispiels 1
20,7 g (0,15 val) para-Hydroxybenzoesäure.
Durchführung wie in Beispiel 9.
Ausbeute: 297,0 g, das sind 95,8 Gew.-% der Theorie, an erfindungsgemäßer Verbindung in Form eines wachsartigen, braun gefärbten Produktes. Die Bruttozusammensetzung der erfindungsgemäßen Verbindung mit einem aliphatischen Fluoralkohol, Isocyanat und para-Hydroxybenzoesäure im Molekül (Molverhältnis 2 : 1 : 1) entspricht der Formel B 12.

T A B E L L E

| Nr. | Chemische Formeln der erfindungsgemäßen Verbindungen nach den Beispielen 1 bis 12 |
| --- | --- |
| B1 | 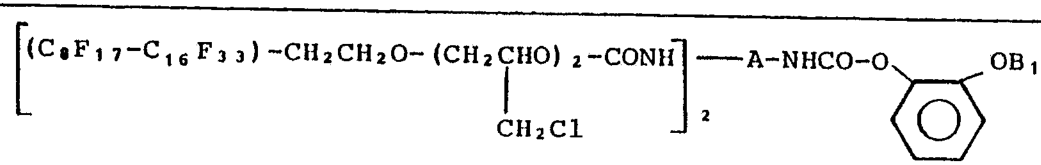 |
| B2 | 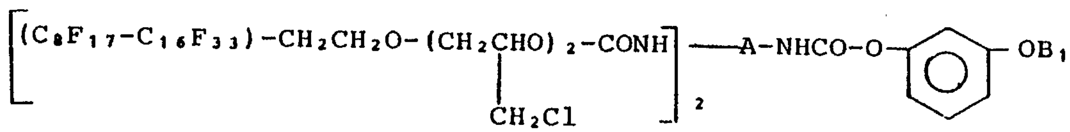 |
| B3 | 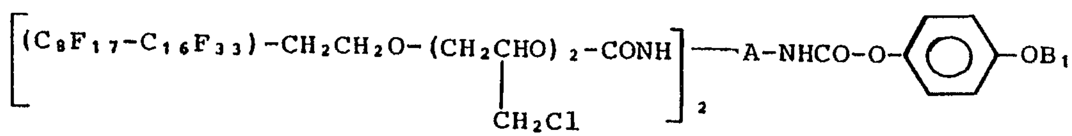 |
| B4 | 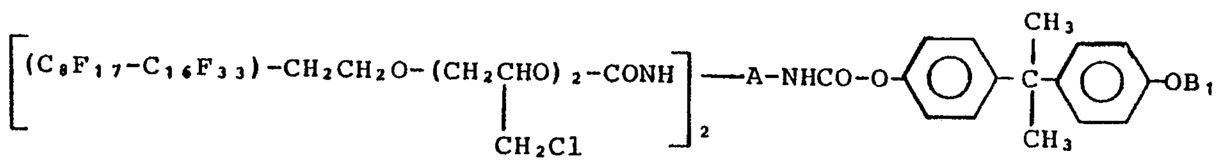 |
| B5 | 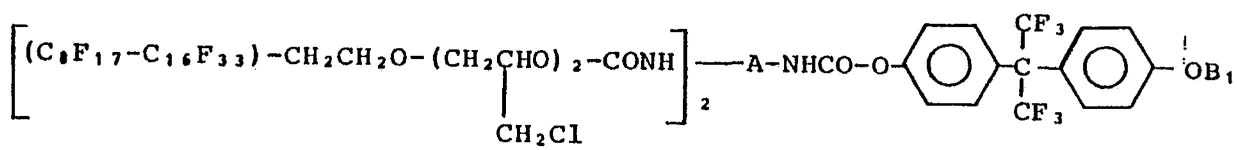 |
| B6 | 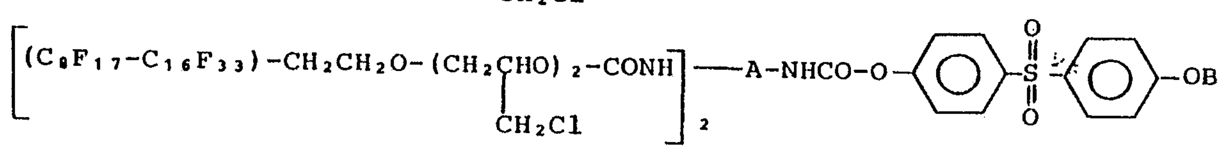 |

T A B E L L E (fortgesetzt)

| Nr. | Chemische Formeln der erfindungsgemäßen Verbindungen nach den Beispielen 1 bis 12 |
|---|---|
| B7 | 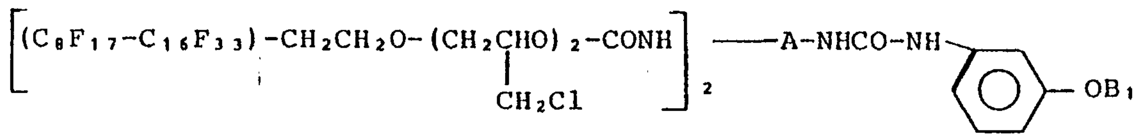 |
| B8 | 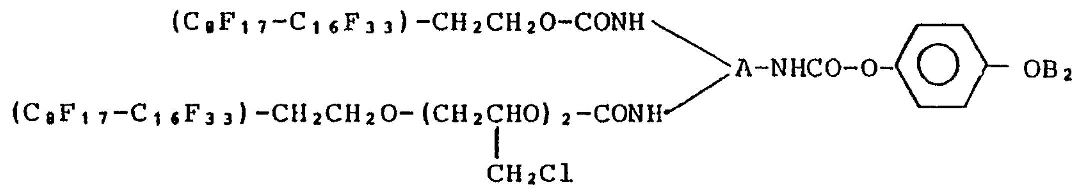 |
| B9 | 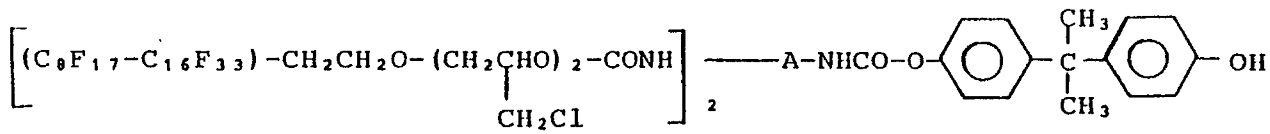 |
| B10 | 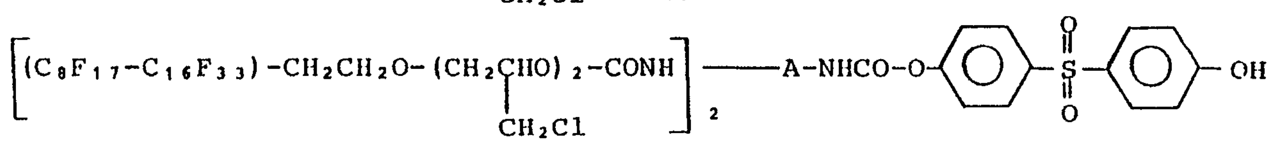 |
| B11 | 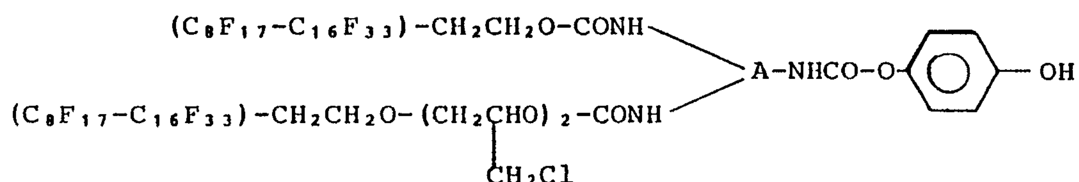 |
| B12 | 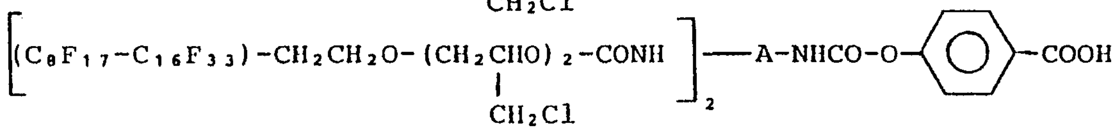 |

In den Formeln B1 bis B 12 steht

A für

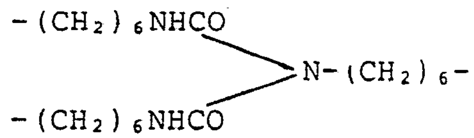 (vgl. obengenannte Formel 10).

In den Formeln B1 bis B7 steht $B_1$ für

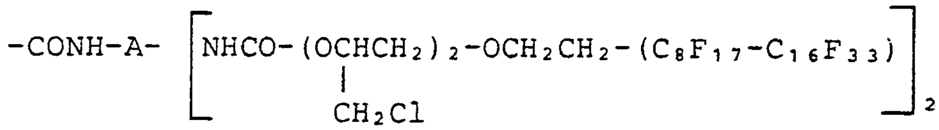 .

In der Formel B8 steht $B_2$ für

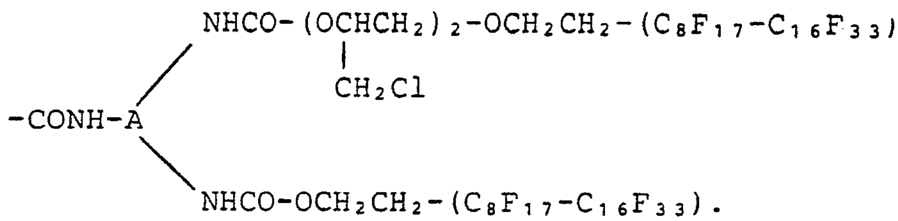

Verwendung der erfindungsgemäßen Verbindungen

Beispiele I bis XII

In den Beispielen I bis XII wurden die erfindungsgemäßen Verbindungen B1 bis B12 mit Hilfe einer üblichen Spinnpräparation für Polyamidfasern getestet, die jeweils ca. 150 g erfindungsgemäße Verbindung pro 1000 g Spinnpräparation enthielt (die Spinnpräparation bestand also aus Wasser als Hauptkomponente, den üblichen ethoxylierten Fettalkoholen und langkettigen Aminoxiden als Präparationsmittel und ca. 15 Gew.-% erfindungsgemäßer Verbindung). Mit jeder der zwölf Spinnpräparationen wurden jeweils gleiche Polyamid-6-Filamente behandelt, um soviel von der erfindungsgemäßen Verbindung und dem Präparationsmittel auf die Filamente aufzubringen, daß 0,05 Gew.-% Fluor und 1 Gew.-% Präparationsmittel auf den Filamenten vorlagen, Gewichtsprozente jeweils bezogen auf das Gewicht der Filamente. Dazu wurden die Filamente in üblicher Weise durch die Spinnpräparation gezogen, getrocknet und 30 Sekunden lang bei einer Temperatur von 200 °C gehalten (Wärmebehandlung, Kondensation). Aus den so behandelten Filamenten wurde jeweils ein Gewebe hergestellt. Es lagen zwölf Gewebe mit den erfindungsgemäßen Verbindungen B1 bis B12 vor, wobei auf jedem Gewebe eine Fluorauflage von 0,05 Gew.-% und eine Präparationsmittelauflage von 1 Gew.-% vorhanden waren, Gewichtsprozente jeweils bezogen auf das Gewicht des Gewebes.
An den zwölf Geweben wurden die Ölabweisung (Oleophobie) nach der AATCC-Prüfnorm 118 - 1966 und die Wasserabweisung (Hydrophobie) nach DIN 53 888 - 1965 geprüft, und zwar nach der beschriebenen Kondensation und nach einer dreistündigen Behandlung der kondensierten Gewebe mit einer alkalischen Kochwäsche. Bei dieser Behandlung wurden die einzelnen Gewebe in üblicher Weise 3 Stunden lang in einer alkalischen Waschflüssigkeit gekocht und anschließend getrocknet; die Waschflüssigkeit bestand aus 1 l Wasser, 1 g Trinatriumphosphat und 2 g eines Fettsäurepolyglykolesters, der durch Oxethylierung von Butandiol-1,4 mit 15 mol Ethylenoxid und anschließender Veresterung des Oxethylats mit 1 mol Ölsäure erhalten worden ist.
Die Ergebnisse aus den Beispielen I bis XII sind nachstehend zusammengefaßt:

| Beispiele und getestete Verbindungen | Ölabweisung nach der Konden-sation | nach der Koch-wäsche | Wasserabweisung nach der Konden-sation | nach der Koch-wäsche |
|---|---|---|---|---|
| I /B1 | 6 | 3 | 5 | 4 |
| II /B2 | 6 | 3 | 5 | 4 |
| III/B3 | 6 | 4 | 5 | 4 |
| IV /B4 | 5 | 4 | 4 | 4 |
| V /B5 | 5 | 4 | 4 | 4 |
| VI /B6 | 5 | 4 | 5 | 4 |
| VII/B7 | 5 | 4 | 5 | 4 |
| VIII/B8 | 6 | 5 | 5 | 4 |
| IX /B9 | 5 | 5 | 5 | 4 |
| X /B10 | 5 | 4 | 4 | 3 |
| XI /B11 | 5 | 4 | 4 | 3 |
| XII/B12 | 5 | 4 | 5 | 4 |

Im folgenden wird der AATCC-Test 118 - 1966 (American Association of Textile Chemists and Colorists) und DIN 53 888 - 1965 (Deutsche Industrie-Norm) beschrieben: Zur Bestimmung des Ölabweisungswertes nach AATCC-Test 118 - 1966 werden bekanntlich drei Tropfen von einer bestimmten Testflüssigkeit (siehe unten) vorsichtig auf das zu prüfende Textilmaterial aufgelegt.
Einwirkungszeit: 30 Sekunden. Es wird der Wert angegeben, bei dem noch keine augenscheinliche Benetzung des Gewebes unter den Tropfen (nach abgelaufener Einwirkungszeit) hervorgerufen worden ist:

| Testflüssigkeit | Ölabweisungswert |
|---|---|
| Paraffinöl | 1 |
| Paraffinöl : n-Hexadecan = 65 : 35 | 2 |
| n-Hexadecan | 3 |
| n-Tetradecan | 4 |
| n-Dodecan | 5 |
| n-Decan | 6 |
| n-Octan | 7 |
| n-Heptan | 8 |

Ein Ölabweisungswert von 1 bedeutet den schlechtesten und ein Ölabweisungswert von 8 den besten Abweisungseffekt.

Zur Bestimmung des Wasserabweisungswertes nach DIN 53 888 - 1965 werden bekanntlich die zu prüfenden Textilien unter standardisierten Bedingungen beregnet, wobei gleichzeitig die Unterseite der Textilprobe mechanisch gerieben wird. Es wird der Wasserabperleffekt visuell mit den Noten 1 bis 5 beurteilt, wobei Note 1 den schlechtesten und Note 5 den besten Abperleffekt bedeutet.

Die Testergebnisse zeigen, daß mit den erfindungsgemäßen Urethanen eine sehr hohe Öl- und Wasserabweisung erreicht wird, und daß die erfindungsgemäßen Urethane auch Textilbehandlungspräparationen zugesetzt werden können.

**Ansprüche**

Patentansprüche für folgende Vertragsstaaten: AT, BE, CH, DE, FR, GB, IT, LI, NL

1. Urethane aus aliphatischen Fluoralkoholen, Isocyanaten und aromatischen Verbindungen der nachstehenden allgemeinen Formel 1

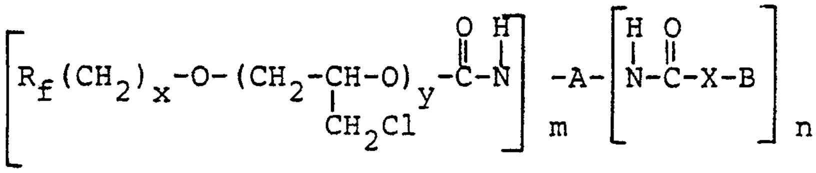 (1)

worin bedeuten:

- $R_f$ eine Perfluoralkylgruppe mit 4 bis 20 C-Atomen oder eine $R'_fSO_2NR_1$-Gruppe, in der $R'_f$ eine der Bedeutungen von $R_f$ hat und $R_1$ H oder eine Alkylgruppe mit 1 bis 4 C-Atomen ist,
- x eine ganze Zahl von 1 bis 4,
- y eine Zahl von 0 bis 10,
- m eine Zahl von 1 bis 2 und
- n eine Zahl von 1 bis 2, wobei die Summe aus m + n höchstens 3 ist,
- A eine der Gruppen entsprechend den nachstehenden Formeln 2 bis 10:

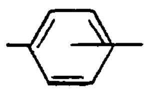 (2)

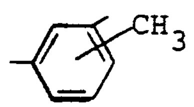 (3)

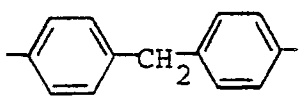 (4)

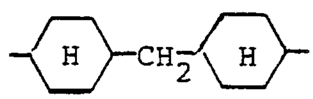 (5)

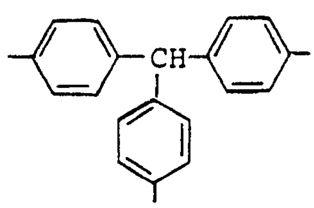 (6)

$-(CH_2)_6-$ (7)

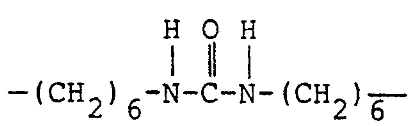 (8)

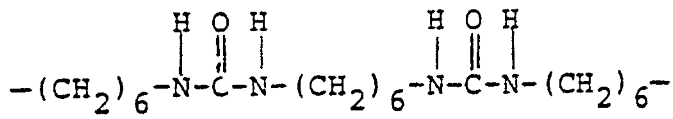

(9)

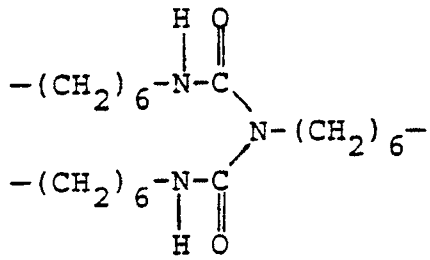

(10)

X eine der Gruppen entsprechend den nachstehenden Formeln 11 bis 19, die mit einer Alkylgruppe mit 1 bis 4 C-Atomen ein- oder mehrfach substituiert sein können:

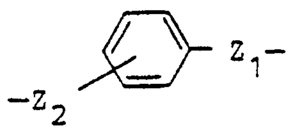

(11)

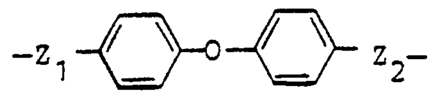

(12)

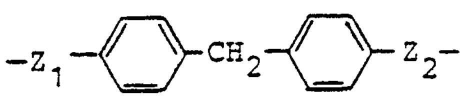

(13)

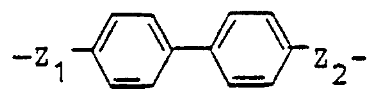

(14)

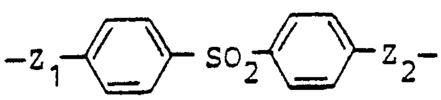

(15)

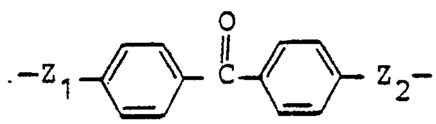

(16)

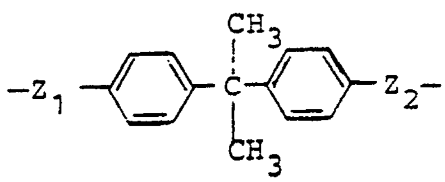

(17)

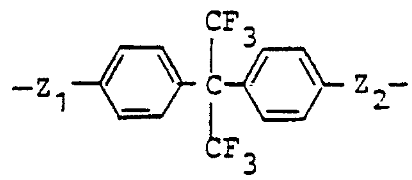

(18)

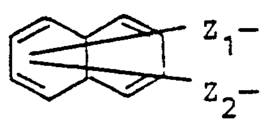

(19) ,

worin $Z_1$ und $Z_2$ für O, NH oder COO stehen, wobei die beiden Substituenten nicht gleich sind, oder für O oder NH stehen, wobei die beiden Substituenten auch gleich sein können, und

B das Wasserstoffatom, eine Alkylgruppe mit 1 bis 4 C-Atomen oder eine Gruppe entsprechend der nachstehenden Formel 20

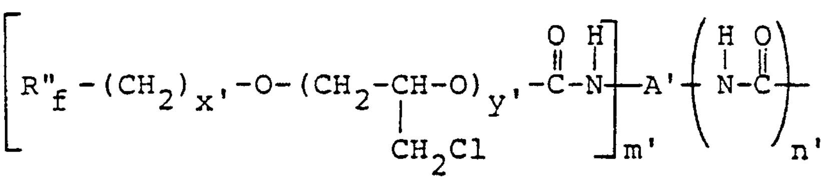

(20) ,

worin $R''_f$, x', y', m', n' und A' eine der Bedeutungen von $R_f$, x, y, m, n und A haben.

**2.** Urethane nach Anspruch 1, wobei bedeuten:

$R_f$ eine Perfluoralkylgruppe mit 6 bis 16 C-Atomen,
x 2,
y 1 bis 5,
m 1 bis 2,
n 1 bis 2, wobei die Summe m + n höchstens 3 ist,
A eine Toluylengruppe oder eine der drei Gruppen entsprechend den Formeln 8 bis 10,
X eine Gruppe entsprechend den Formeln 11, 15, 16, 17 oder 18, und
B H oder eine Gruppe entsprechend der Formel 20, wobei A' eine der drei Gruppen entsprechend den Formeln 8 bis 10 ist.

3. Urethane nach Anspruch 1, wobei bedeuten:
$R_f$ eine Perfluoralkylgruppe mit 6 bis 16 C-Atomen,
x 2,
y 1 bis 5,
m 1 bis 2,
n 1 bis 2, wobei die Summe von m + n höchstens 3 ist,
A eine Toluylengruppe oder eine der drei Gruppen entsprechend den Formeln 8 bis 10,
X eine Gruppe entsprechend den Formeln 11, 15, 16, 17 oder 18, und
B H oder eine Gruppe entsprechend der Formel 20, wobei A' eine der drei Gruppen entsprechend den Formeln 8 bis 10 ist, $R''_f$ eine Perfluoralkylgruppe mit 6 bis 16 C-Atomen, x' 2, y' 1 bis 5, m' 1 bis 2 und n' 1 bis 2, wobei die Summe m' + n' höchstens 3 ist.

4. Verfahren zur Herstellung der Urethane nach Anspruch 1, gekennzeichnet durch Reaktion eines aliphatischen Fluoralkohols der Formel

$$R_f(CH_2)_xO(\underset{\substack{|\\CH_2Cl}}{CH_2CHO})_y\text{-}H \quad ,$$

worin $R_f$, x und y die obengenannte Bedeutung haben,
mit einem Di- oder Triisocyanat entsprechend einer der Gruppen der Formeln 2 bis 10 zum Addukt der Formel

$$\left[R_f(CH_2)_xO(\underset{\substack{|\\CH_2Cl}}{CH_2CHO})_y\text{-}CONH\right]_m\!\!\text{—}A\text{-}(NCO)_n \quad \text{(Addukt 1)},$$

worin $R_f$, x, y, m, n und A die obengenannte Bedeutung haben,
und durch Reaktion des Adduktes 1 mit einer aromatischen Dihydroxy-, Diamino-, Aminohydroxy-, Aminocarboxy-oder Hydroxycarboxy-Verbindung der nachstehenden Formeln

H-X-H oder H-X-B ,

worin X eine der Gruppen der Formeln 11 bis 19 und B eine Alkylgruppe mit 1 bis 4 C-Atomen ist,
zu den angestrebten Urethanen der beiden nachstehenden Formeln 21 und 22

$$\left[R_f(CH_2)_xO(CH_2\underset{\substack{|\\ CH_2Cl}}{C}HO)_y-CONH\right]_m-A-\left[NHCO-X-H\right]_n \qquad (21)$$

$$\left[R_f(CH_2)_xO(CH_2\underset{\substack{|\\ CH_2Cl}}{C}HO)_y-CONH\right]_m-A-\left[NHCO-X-B\right]_n \qquad (22),$$

worin $R_f$, x, y, m, n und A die obengenannte Bedeutung haben und B die genannte Alkylgruppe mit 1 bis 4 C-Atomen ist, und durch Reaktion eines Urethans der Formel 21 mit einer eine oder mehrere freie Isocyanatgruppen enthaltenden Isocyanat-Verbindung entsprechend der Gruppe der Formel 20 zu den angestrebten Urethanen der nachstehenden Formel 23

$$\left[R_f(CH_2)_xO(CH_2\underset{\substack{|\\ CH_2Cl}}{C}HO)_y-CONH\right]_m-A-\left\{NHCO-X-(CONH)_{n'}-A'-\left[NHCO-(O\underset{\substack{|\\ CH_2Cl}}{C}HCH_2)_{y'}-O(CH_2)_{x'}-R''_f\right]_{m'}\right\}_n \qquad (23),$$

worin $R_f$, $R''_f$, x, x', y, y', m, m', n, n', A, A' und X die obengenannte Bedeutung haben.

5. Verwendung der Urethane nach einem oder mehreren der Ansprüche 1 bis 3 zur Oleophob- und Hydrophob-Ausrüstung von Textilien und Leder.

Patentansprüche für folgenden Vertragsstaat: ES

1. Verfahren zur Herstellung von Urethanenaus aliphatischen Fluoralkoholen, Isocyanaten und aromatischen Verbindungen der nachstehenden allgemeinen Formel 1

$$\left[R_f(CH_2)_x-O-(CH_2-\underset{\substack{|\\ CH_2Cl}}{C}H-O)_y-\overset{\substack{O\\ \|}}{C}-\overset{\substack{H\\ |}}{N}\right]_m-A-\left[\overset{\substack{H\\ |}}{N}-\overset{\substack{O\\ \|}}{C}-X-B\right]_n \qquad (1)$$

worin bedeuten:

- $R_f$ eine Perfluoralkylgruppe mit 4 bis 20 C-Atomen oder eine $R'_fSO_2NR_1$-Gruppe, in der $R'_f$ eine der Bedeutungen von $R_f$ hat und $R_1$ H oder eine Alkylgruppe mit 1 bis 4 C-Atomen ist,
- x eine ganze Zahl von 1 bis 4,
- y eine Zahl von 0 bis 10,
- m eine Zahl von 1 bis 2 und
- n eine Zahl von 1 bis 2, wobei die Summe aus m + n höchstens 3 ist,
- A eine der Gruppen entsprechend den nachstehenden Formeln 2 bis 10:

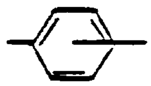 (2)

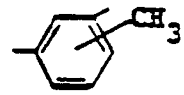 (3)

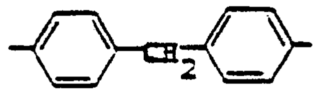 (4)

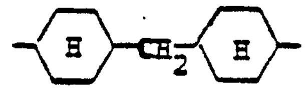 (5)

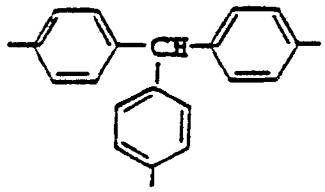 (6)

$-(CH_2)_6-$ (7)

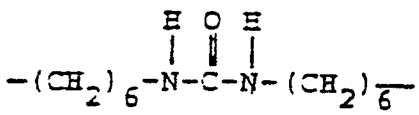 (8)

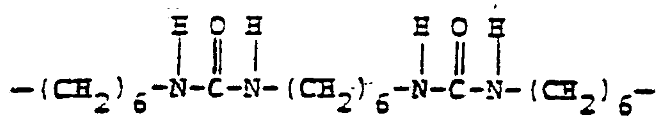 (9)

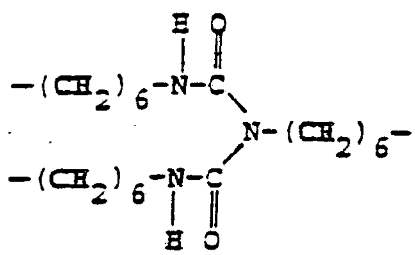 (10)

X eine der Gruppen entsprechend den nachstehenden Formeln 11 bis 19, die mit einer Alkylgruppe mit 1 bis 4 C-Atomen ein- oder mehrfach substituiert sein können:

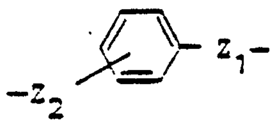 (11)

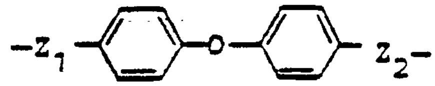 (12)

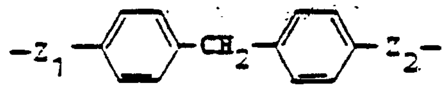 (13)

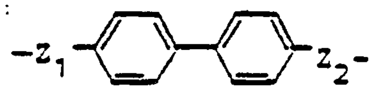 (14)

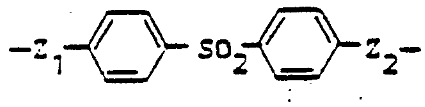 (15)

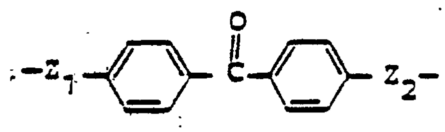 (16)

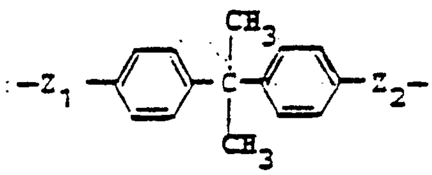 (17)

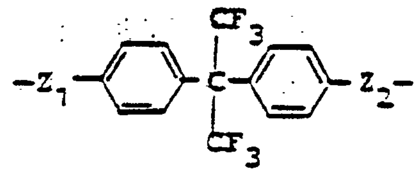 (18)

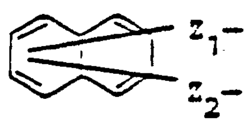 (19) ,

worin $Z_1$ und $Z_2$ für O, NH oder COO stehen, wobei die beiden Substituenten nicht gleich sind, oder für O oder NH stehen, wobei die beiden Substituenten auch gleich sein können, und

B das Wasserstoffatom, eine Alkylgruppe mit 1 bis 4 C-Atomen oder eine Gruppe entsprechend der nachstehenden Formel 20

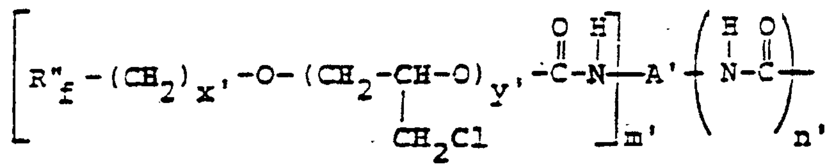 (20) ,

worin $R''_f$, x', y', m', n' und A' eine der Bedeutungen von $R_f$, x, y, m, n und A haben, gekennzeichnet durch Reaktion eines aliphatischen Fluoralkohols der Formel

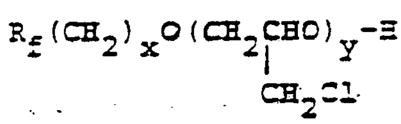 ,

worin $R_f$, x und y die obengenannte Bedeutung haben,
mit einen Di- oder Triisocyanat entsprechend einer der Gruppen der Formeln 2 bis 10 zum Addukt der Formel

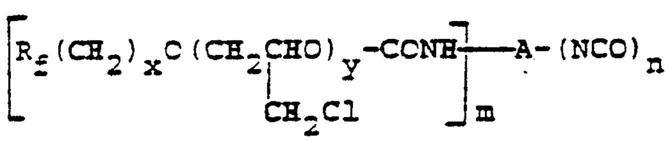 (Addukt 1) ,

worin $R_f$, x, y, m, n und A die obengenannte Bedeutung haben,
und durch Reaktion des Adduktes 1 mit einer aromatischen Dihydroxy-, Diamino-, Aminohydroxy-, Aminocarboxy-oder Hydroxycarboxy-Verbindung der nachstehenden Formeln

H-X-H oder H-X-B ,

worin X eine der Gruppen der Formeln 11 bis 19 und B eine Alkylgruppe mit 1 bis 4 C-Atomen ist,
zu den angestrebten Urethanen der beiden nachstehenden Formeln 21 und 22

$$\left[R_f(CH_2)_xO(CH_2\underset{\displaystyle CH_2Cl}{\underset{|}{C}}HO)_y-CONH\right]_m-A-\left[NHCO-X-H\right]_n \qquad (21)$$

$$\left[R_f(CH_2)_xO(CH_2\underset{\displaystyle CH_2Cl}{\underset{|}{C}}HO)_y-CONH\right]_m-A-\left[NHCO-X-B\right]_n \qquad (22),$$

worin $R_f$, x, y, m, n und A die obengenannte Bedeutung haben und B die genannte Alkylgruppe mit 1 bis 4 C-Atomen ist,
und durch Reaktion eines Urethans der Formel 21 mit einer eine oder mehrere freie Isocyanatgruppen enthaltenden Isocyanat-Verbindung entsprechend der Gruppe der Formel 20 zu den angestrebten Urethanen der nachstehenden Formel 23

$$\left[R_f(CH_2)_xO(CH_2\underset{\displaystyle CH_2Cl}{\underset{|}{C}}HO)_y-CONH\right]_m-A-\Big\{NHCO-X-(CONH)_{n'}-A'-\left[NHCO-(O\underset{\displaystyle CH_2Cl}{\underset{|}{C}}HCH_2)_{y'}-O(CH_2)_{x'}-R''_f\right]_{m'}\Big\}_n \qquad (23),$$

worin $R_f$, $R''_f$, x, x′, y, y′, m, m′, n, n′, A, A′ und X die obengenannte Bedeutung haben.

2. Verfahren nach Anspruch 1, dadurch gekennzeichnet, daß zur Reaktion Verbindungen eingesetzt werden, die sich ergeben,wenn
   - $R_f$ eine Perfluoralkylgruppe mit 6 bis 16 C-Atomen ist,
   - x 2,
   - y 1 bis 5,
   - m 1 bis 2,
   - n 1 bis 2,wobei die Summe m + n höchstens 3 ist,
   - A eine Toluylengruppe oder eine der drei Gruppen entsprechend den Formeln 8 bis 10,
   - X eine Gruppe entsprechend den Formeln 11, 15, 16, 17 oder 18, und
   - B H oder eine Gruppe entsprechend der Formel 20 ist, wobei A′ eine der drei Gruppen entsprechend den Formeln 8 bis 10 ist.

3. Verfahren nach Anspruch 1, dadurch gekennzeichnet, daß zur Reaktion Verbindungen eingesetzt werden, die sich ergeben, wenn
   - $R_f$ eine Perfluoralkylgruppe mit 6 bis 16 C-Atomen ist,
   - x 2,
   - y 1 bis 5,

m 1 bis 2,
n 1 bis 2, wobei die Summe von m + n höchstens 3 ist,
A eine Toluylengruppe oder eine der drei Gruppen entsprechend den Formeln 8 bis 10,
X eine Gruppe entsprechend den Formeln 11, 15, 16, 17 oder 18, und
B H oder eine Gruppe entsprechend der Formel 20 ist, wobei A'eine der drei Gruppen entsprechend den Formeln 8 bis 10 ist, $R_f''$ eine Perfluoralkylgruppe mit 6 bis 16 C-Atomen, x' 2, y' 1 bis 5, m' 1 bis 2 und n' 1 bis 2, wobei die Summe m' + n' höchstens 3 ist.

**4.** Verwendung der nach dem Verfahren der Ansprüche 1 bis 3 hergestellten Urethane zur oleophoben und hydrophoben Ausrüstung von Textilien und Leder.

**Claims**

Claims for the following Contracting States: AT, BE, CH, DE, FR, GB, IT, LI, NL

**1.** A urethane formed from aliphatic fluoroalcohols, isocyanates and aromatic compounds and having the formula 1 below

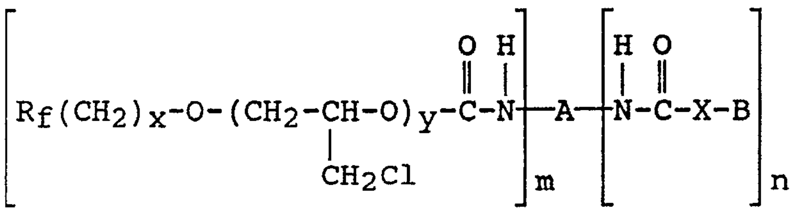 (1)

in which

$R_f$ denotes a perfluoroalkyl group having 4 to 20 carbon atoms, or an $R'_fSO_2NR_1$ group in which $R'_f$ has one of the meanings of $R_f$, and $R_1$ is H or an alkyl group having 1 to 4 carbon atoms,
x denotes an integer from 1 to 4,
y denotes a number from 0 to 10,
m denotes a number from 1 to 2 and
n denotes a number from 1 to 2, the sum of m + n being not more than 3,
A denotes one of the groups corresponding to the formulae 2 to 10 below

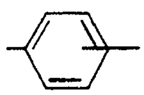 (2)

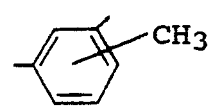 (3)

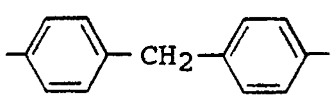 (4)

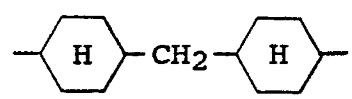 (5)

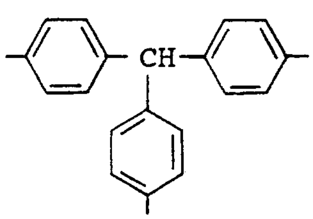 (6)

$-(CH_2)_6-$ (7)

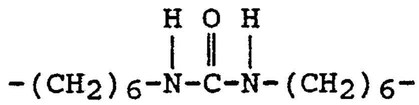 (8)

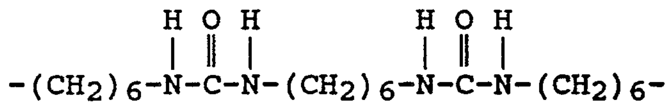 (9)

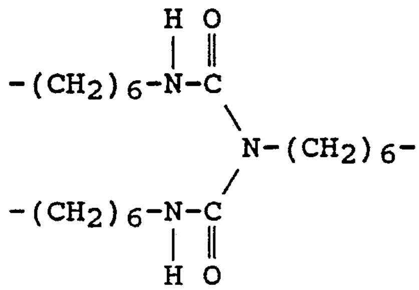 (10)

X denotes one of the groups corresponding to the formulae 11 to 19 below, which can be monosubstituted or polysubstituted by an alkyl group having 1 to 4 carbon atoms

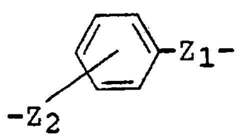 (11)

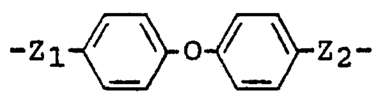 (12)

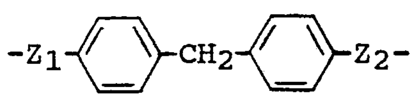 (13)

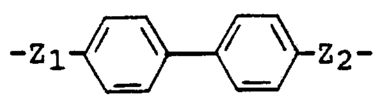 (14)

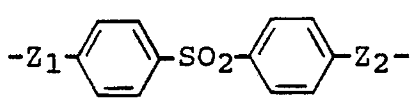 (15)

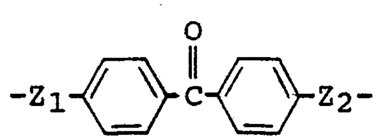 (16)

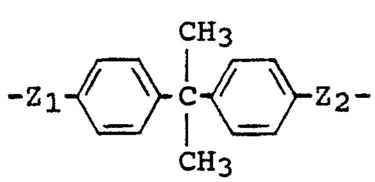 (17)

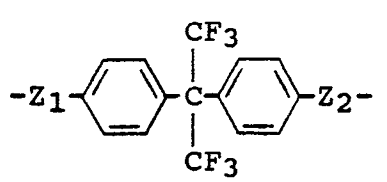 (18)

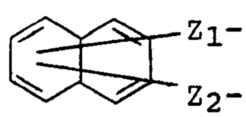 (19)

in which $Z_1$ and $Z_2$ represent O, NH, or COO in which the two substituents are not identical, or represent O or NH in which the two substituents can also be identical, and

B denotes a hydrogen atom, an alkyl group having 1 to 4 carbon atoms or a group corresponding to the formula 20 below

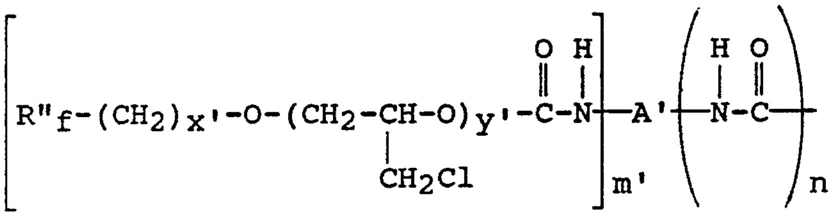

(20)

in which $R''_f$, x', y', m', n', and A' have one of the meanings of $R_f$, x, y, m , n, and A.

**2.** A urethane as claimed in claim 1, in which

- $R_f$ denotes a perfluoroalkyl group having 6 to 16 carbon atoms,
- x denotes 2,
- y denotes 1 to 5,
- m denotes 1 to 2,
- n denotes 1 to 2, the sum of m + n being not more than 3,
- A denotes a toluylene group or one of the three groups corresponding to the formulae 8 to 10,
- X denotes a group corresponding to the formulae 11, 15, 16, 17, or 18 and
- B denotes H or a group corresponding to the formula 20 in which A' is one of the three groups corresponding to the formulae 8 to 10.

**3.** A urethane as claimed in claim 1, in which

- $R_f$ denotes a perfluoroalkyl group having 6 to 16 carbon atoms,
- x denotes 2,
- y denotes 1 to 5,
- m denotes 1 to 2,
- n denotes 1 to 2, the sum of m + n being not more than 3,
- A denotes a toluylene group or one of the three groups corresponding to the formulae 8 to 10,
- X denotes a group corresponding to the formulae 11, 15, 16, 17, or 18 and
- B denotes H or a group corresponding to the formula 20 in which A' is one of the three groups corresponding to the formulae 8 to 10, $R''_f$ is a perfluoroalkyl group having 6 to 16 carbon atoms, x' is 2, y' is 1 to 5, m' is 1 to 2, and n' is 1 to 2, the sum of m' + n' being not more than 3.

**4.** A process for the preparation of a urethane as claimed in claim 1, characterized by reacting an aliphatic fluoroalcohol of the formula

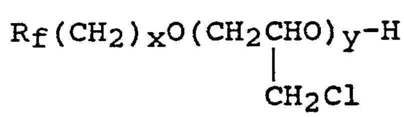

in which $R_f$, x, and y have the above-mentioned meaning,
with a diisocyanate or triisocyanate corresponding to one of the groups of the formulae 2 to 10 to give the adduct of the formula

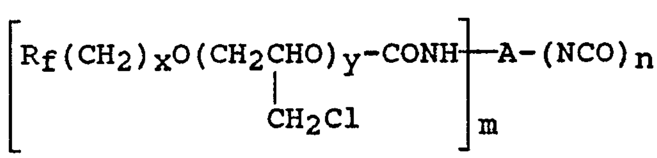

(adduct 1)

in which $R_f$, x, y, m, n, and A have the above-mentioned meaning,
and by reacting the adduct 1 with an aromatic dihydroxy, diamino, aminohydroxy, aminocarboxy, or hydroxycarboxy compound of the following formulae

H-X-H or H-X-B

in which X is one of the groups of the formulae 11 to 19 and B is an alkyl group having 1 to 4 carbon atoms,
to give the desired urethanes of the two formulae 21 and 22 below

$$\left[R_f(CH_2)_xO(CH_2\underset{\displaystyle CH_2Cl}{\underset{|}{C}}HO)_y\text{-}CONH\right]_m\!\!\!-A-\!\!\left[NHCO\text{-}X\text{-}H\right]_n \qquad (21)$$

$$\left[R_f(CH_2)_xO(CH_2\underset{\displaystyle CH_2Cl}{\underset{|}{C}}HO)_y\text{-}CONH\right]_m\!\!\!-A-\!\!\left[NHCO\text{-}X\text{-}B\right]_n \qquad (22)$$

in which $R_f$, x, y, m, n, and A have the above-mentioned meaning and B is the alkyl group mentioned having 1 to 4 carbon atoms,
and by reacting a urethane of the formula 21 with an isocyanate compound containing one or more free isocyanate groups and corresponding to the group of the formula 20, to give the desired urethanes of the formula 23 below

$$\left[R_f(CH_2)_xO(CH_2\underset{\displaystyle CH_2Cl}{\underset{|}{C}}HO)_y\text{-}CONH\right]_m\!\!\!-A-\left\{NHCO\text{-}X\text{-}(CONH)_{n'}\text{-}A'-\left[NHCO\text{-}(O\underset{\displaystyle CH_2Cl}{\underset{|}{C}}HCH_2)_{y'}\text{-}O(CH_2)_{x'}\text{-}R''_f\right]_{m'}\right\}_n \qquad (23)$$

in which $R_f$, $R''_f$, x, x', y, y', m, m', n, n', A, A', and X have the above-mentioned meaning.

5. The use of a urethane as claimed in one or more of claims 1 to 3 for imparting an oleophobic and hydrophobic finish to textiles and leather.

Claims for the following Contracting State: ES

1. A process for the preparation of a urethane formed from aliphatic fluoroalcohols, isocyanates and aromatic compounds and having the formula 1 below

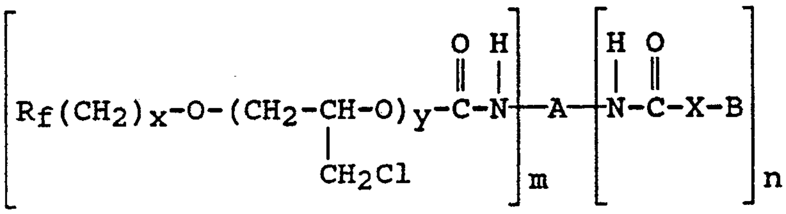 (1)

in which

- $R_f$ denotes a perfluoroalkyl group having 4 to 20 carbon atoms, or an $R'_fSO_2NR_1$ group in which $R'_f$ has one of the meanings of $R_f$, and $R_1$ is H or an alkyl group having 1 to 4 carbon atoms,
- x denotes an integer from 1 to 4,
- y denotes a number from 0 to 10,
- m denotes a number from 1 to 2 and
- n denotes a number from 1 to 2, the sum of m + n being not more than 3,
- A denotes one of the groups corresponding to the formulae 2 to 10 below

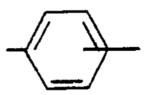 (2)

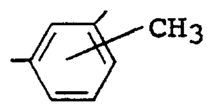 (3)

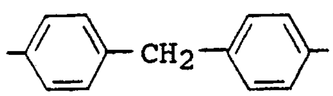 (4)

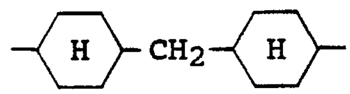 (5)

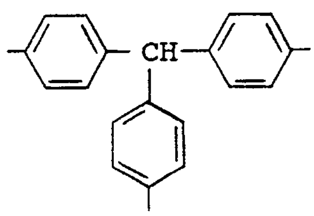 (6)

$-(CH_2)_6-$ (7)

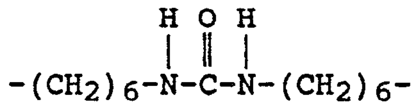

(8)

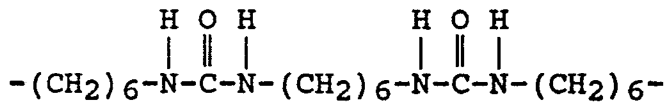

(9)

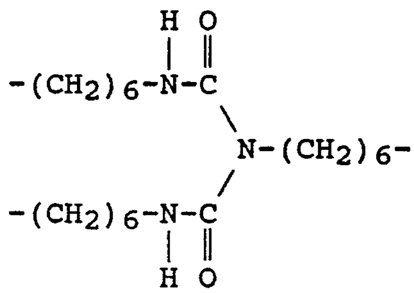

(10)

X denotes one of the groups corresponding to the formulae 11 to 19 below, which can be monosubstituted or polysubstituted by an alkyl group having 1 to 4 carbon atoms

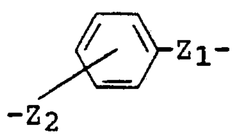

(11)

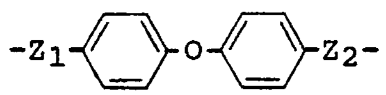

(12)

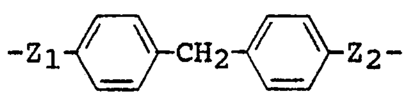

(13)

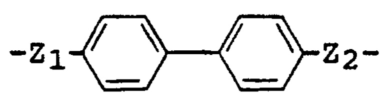

(14)

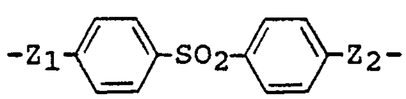

(15)

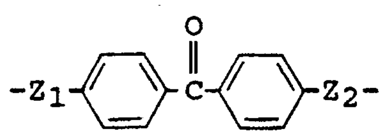

(16)

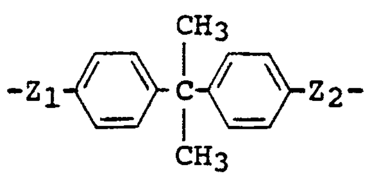

(17)

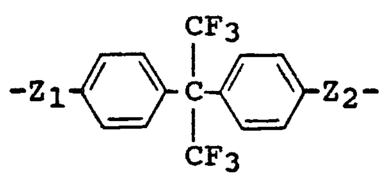

(18)

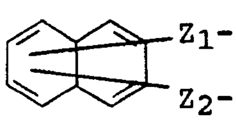

(19)

in which $Z_1$ and $Z_2$ represent O, NH, or COO in which the two substituents are not identical, or represent O or NH in which the two substituents can also be identical, and

B denotes a hydrogen atom, an alkyl group having 1 to 4 carbon atoms or a group corresponding to the formula 20 below

$$\left[ R''_f\text{-}(CH_2)_{x'}\text{-}O\text{-}(CH_2\text{-}\underset{\displaystyle CH_2Cl}{\underset{|}{CH}}\text{-}O)_{y'}\text{-}\overset{\displaystyle O}{\overset{\|}{C}}\text{-}\overset{\displaystyle H}{\overset{|}{N}} \right]_{m'} A' \left( \overset{\displaystyle H}{\overset{|}{N}}\text{-}\overset{\displaystyle O}{\overset{\|}{C}} \right)_{n'} \qquad (20)$$

in which $R''_f$, x', y', m', n', and A' have one of the meanings of $R_f$, x, y, m , n, and A, characterized by reacting an aliphatic fluoroalcohol of the formula

$$R_f(CH_2)_xO(CH_2\underset{\displaystyle CH_2Cl}{\underset{|}{C}}HO)_y\text{-}H$$

in which $R_f$, x, and y have the above-mentioned meaning, with a diisocyanate or triisocyanate corresponding to one of the groups of the formulae 2 to 10 to give the adduct of the formula

$$\left[ R_f(CH_2)_xO(CH_2\underset{\displaystyle CH_2Cl}{\underset{|}{C}}HO)_y\text{-}CONH \right]_m A\text{-}(NCO)_n \qquad \text{(adduct 1)}$$

in which $R_f$, x, y, m, n, and A have the above-mentioned meaning, and by reacting the adduct 1 with an aromatic dihydroxy, diamino, aminohydroxy, aminocarboxy, or hydroxycarboxy compound of the following formulae

H-X-H or H-X-B

in which X is one of the groups of the formulae 11 to 19 and 8 is an alkyl group having 1 to 4 carbon atoms, to give the desired urethanes of the two formulae 21 and 22 below

$$\left[ R_f(CH_2)_xO(CH_2\underset{\displaystyle CH_2Cl}{\underset{|}{C}}HO)_y\text{-}CONH \right]_m A \left[ NHCO\text{-}X\text{-}H \right]_n \qquad (21)$$

$$\left[ R_f(CH_2)_xO(CH_2\underset{\displaystyle CH_2Cl}{\underset{|}{C}}HO)_y\text{-}CONH \right]_m A \left[ NHCO\text{-}X\text{-}B \right]_n \qquad (22)$$

in which $R_f$, x, y, m, n, and A have the above-mentioned meaning and B is the alkyl group mentioned having 1 to 4 carbon atoms, and by reacting a urethane of the formula 21 with an isocyanate compound containing one or more free isocyanate groups and corresponding to the group of the formula 20, to give the desired urethanes of

the formula 23 below

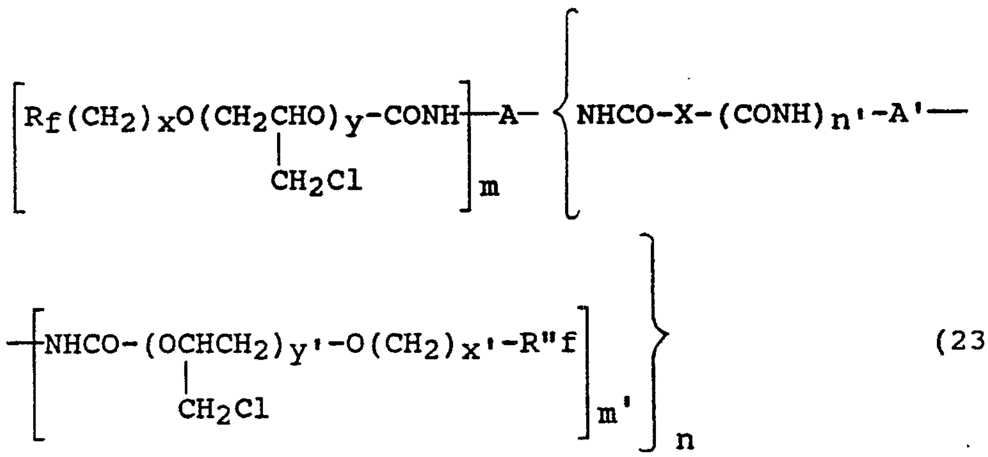

(23)

in which $R_f$, $R''_f$, x, x', y, y', m, m', n, n', A, A', and X have the above-mentioned meaning.

2. The process as claimed in claim 1, wherein the compounds employed for the reaction are those which result if

| | |
|---|---|
| $R_f$ | is a perfluoroalkyl group having 6 to 16 carbon atoms, |
| x | denotes 2, |
| y | denotes 1 to 5, |
| m | denotes 1 to 2, |
| n | denotes 1 to 2, the sum of m + n being not more than 3, |
| A | denotes a toluylene group or one of the three groups corresponding to the formulae 8 to 10, |
| X | denotes a group corresponding to the formulae 11, 15, 16, 17, or 18 and |
| B | denotes H or a group corresponding to the formula 20 in which A' is one of the three groups corresponding to the formulae 8 to 10. |

3. The process as claimed in claim 1, wherein the compounds employed for the reaction are those which result if

| | |
|---|---|
| $R_f$ | is a perfluoroalkyl group having 6 to 16 carbon atoms, |
| x | denotes 2, |
| y | denotes 1 to 5, |
| m | denotes 1 to 2, |
| n | denotes 1 to 2, the sum of m + n being not more than 3, |
| A | denotes a toluylene group or one of the three groups corresponding to the formulae 8 to 10, |
| X | denotes a group corresponding to the formulae 11, 15, 16, 17, or 18 and |
| B | denotes H or a group corresponding to the formula 20 in which A' is one of the three groups corresponding to the formulae 8 to 10, $R''_f$ is a perfluoroalkyl group having 6 to 16 carbon atoms, x' is 2, y' is 1 to 5, m' is 1 to 2, and n' is 1 to 2, the sum of m' + n' being not more than 3. |

4. The use of a urethane prepared as claimed in the process of claims 1 to 3 for imparting an oleophobic and hydrophobic finish to textiles and leather.

## Revendications

Revendications pour les Etats contractants suivants: AT, BE, CH, DE, FR, GB, IT, LI, NL

1. Uréthannes dérivant de fluoro-alcools aliphatiques, d'isocyanates et de composés aromatiques, uréthannes qui répondent à la formule générale 1 :

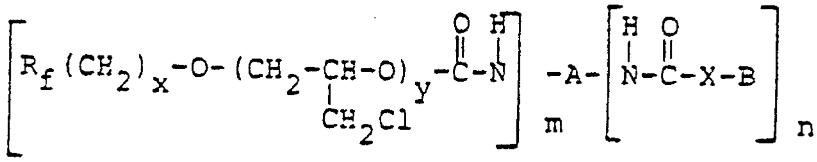 (1)

dans laquelle :

- $R_f$ représente un radical perfluoralkyle contenant de 4 à 20 atomes de carbone, ou un radical $R'_fSO_2NR_1$- dans lequel $R'_f$ a l'une des significations de $R_f$ et $R_1$ représente H ou un alkyle contenant de 1 à 4 atomes de carbone,
- x désigne un nombre entier de 1 à 4,
- y désigne un nombre de 0 à 10,
- m désigne un nombre de 1 à 2,
- n désigne un nombre de 1 à 2, la somme (m + n) étant au plus égale à 3,
- A représente un radical répondant à l'une des formules 2 à 10 suivantes :

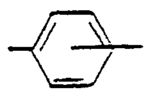 (2)

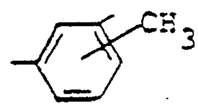 (3)

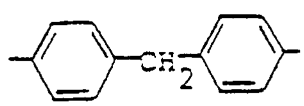 (4)

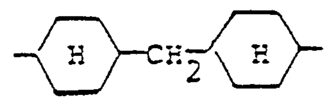 (5)

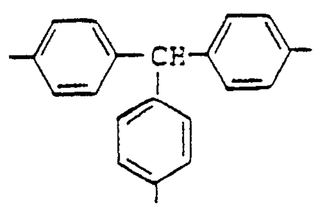 (6)

$-(CH_2)_6-$ (7)

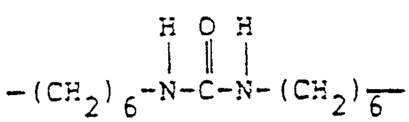 (8)

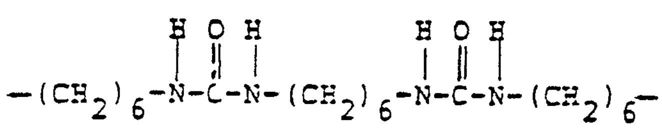 (9)

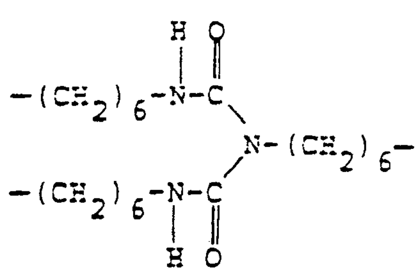 (10)

- X représente un radical répondant à l'une des formules 11 à 19 suivantes, qui peut porter un ou plusieurs alkyles en $C_1$-$C_4$ :

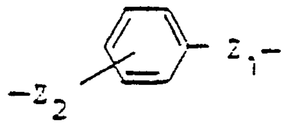 (11) 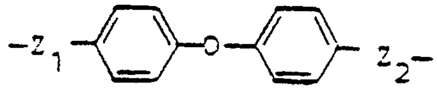 (12)

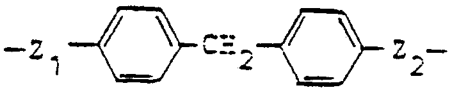 (13) 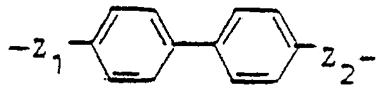 (14)

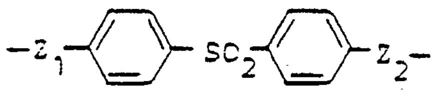 (15) 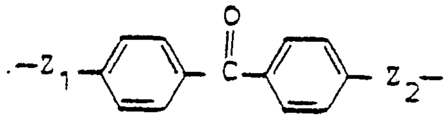 (16)

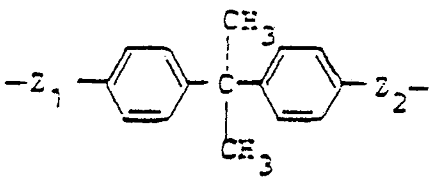 (17) 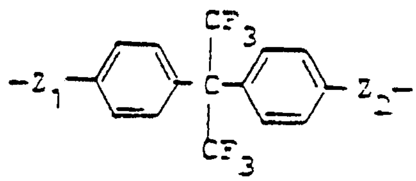 (18)

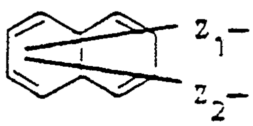 (19) ,

dans lesquelles $Z_1$ et $Z_2$ représentent chacun O, NH ou COO, auquel cas les deux substituants ne sont pas identiques, ou représentent chacun O ou NH, auquel cas les deux substituants peuvent également être identiques,

et

B représente un atome d'hydrogène, un radical alkyle contenant de 1 à 4 atomes de carbone ou un radical répondant à la formule 20 :

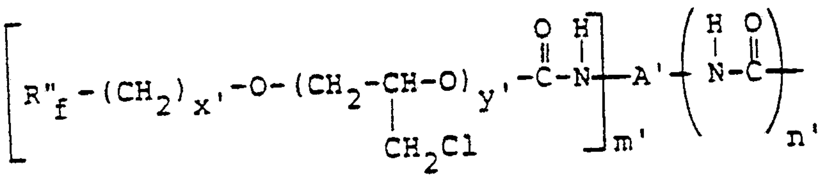 (20)

dans laquelle $R''_f$, x', y', m', n' et A' ont chacun une des significations de $R_f$, x, y, m, n et A.

**2.** Uréthannes selon la revendication 1 dans lesquels :

- $R_f$ représente un radical perfluoralkyle contenant de 6 à 16 atomes de carbone,
- x est égal à 2,
- y désigne un nombre de 1 à 5,
- m désigne un nombre de 1 à 2,
- n désigne un nombre de 1 à 2, la somme (m + n) étant au plus égale à 3,
- A représente un radical toluylène, ou un radical répondant à l'une des trois formules 8 à 10,
- X représente un radical répondant à l'une des formules 11, 15, 16, 17 et 18, et
- B représente H ou un radical de formule 20 dans lequel A' représente un radical répondant à l'une des trois formules 8 à 10.

**3.** Uréthannes selon la revendication 1 dans lesquels :

- $R_f$ représente un radical perfluoralkyle contenant de 6 à 16 atomes de carbone,
- x est égal à 2,
- y désigne un nombre de 1 à 5,

m désigne un nombre de 1 à 2,
n désigne un nombre de 1 à 2, la somme (m + n) étant au plus égale à 3,
A représente un radical toluylène, ou un radical répondant à l'une des trois formules 8 à 10,
X représente un radical répondant à l'une des formules 11, 15, 16, 17 et 18, et
B représente H ou un radical de formule 20 dans lequel A' représente un radical répondant à l'une des trois formules 8 à 10, R"f un radical perfluoralkyle contenant de 6 à 16 atomes de carbone, x' le nombre 2, y' un nombre de 1 à 5, m' un nombre de 1 à 2 et n' un nombre de 1 à 2, la somme (m' + n') étant au plus égale à 3.

4. Procédé pour préparer les uréthannes selon la revendication 1, procédé caractérisé en ce qu'on fait réagir un fluoro-alcool aliphatique répondant à la formule :

$$R_f(CH_2)_xO(CH_2\underset{CH_2Cl}{\underset{|}{C}}HO)_y\text{-}H\ ,$$

dans laquelle $R_f$, x et y ont les significations indiquées ci-dessus,
avec un di- ou tri-isocyanate correspondant à l'un des radicaux de formules 2 à 10, réaction qui conduit à un produit d'addition répondant à la formule :

$$\left[R_f(CH_2)_xO(CH_2\underset{CH_2Cl}{\underset{|}{C}}HO)_y\text{-}CONH\right]_m\text{—}A\text{-}(NCO)_n \qquad \text{(produit d'addition 1)}$$

dans laquelle $R_f$, x, y, m, n et A ont les significations indiquées ci-dessus,
on fait réagir le produit d'addition 1 avec un composé aromatique dihydroxylique, diaminé, amino-hydroxylique, amino-carboxylique ou hydroxy-carboxylique répondant à l'une des formules suivantes :

H-X-H et H-X-B ,

dans lesquelles X représente un radical répondant à l'une des formules 11 à 19 et B un radical alkyle contenant de 1 à 4 atomes de carbone,
réaction qui conduit aux uréthannes voulues répondant aux formules 21 et 22 :

$$\left[R_f(CH_2)_xO(CH_2\underset{CH_2Cl}{\underset{|}{C}}HO)_y\text{-}CONH\right]_m\text{—}A\text{—}\left[NHCO\text{-}X\text{-}H\right]_n \qquad (21)$$

$$\left[R_f(CH_2)_xO(CH_2\underset{CH_2Cl}{\underset{|}{C}}HO)_y\text{-}CONH\right]_m\text{—}A\text{—}\left[NHCO\text{-}X\text{-}B\right]_n \qquad (22),$$

dans lesquelles $R_f$, x, y, m, n et A ont les significations indiquées ci-dessus et B représente l'alkyle en $C_1$-$C_4$ qui vient d'être mentionné,
et on fait réagir un uréthanne de formule 21 avec un composé contenant un ou plusieurs radicaux isocyanato libres qui correspond au radical de formule 20, de manière à obtenir les uréthannes cherchés qui répondent à la formule 23 :

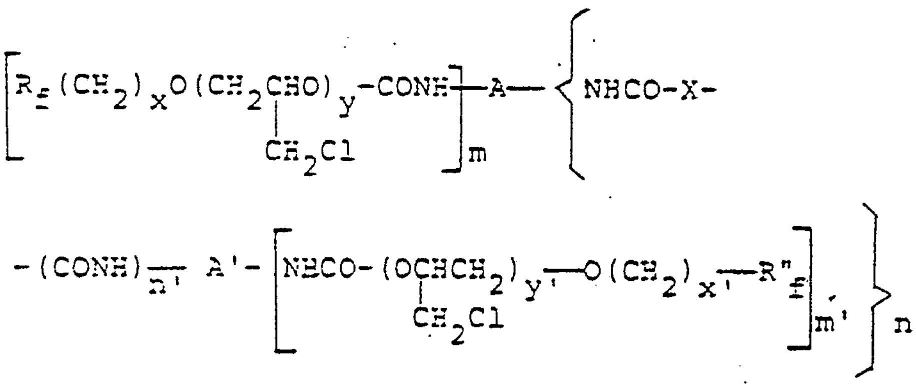

(23),

dans laquelle $R_f$, $R''_f$, x, x', y, y', m, m', n, n', A, A' et X ont les significations qui leur ont été données ci-dessus.

**5.** Application des uréthannes selon l'une quelconque des revendications 1 à 3 pour l'ennoblissement oléophobe et hydrophobe de textiles et du cuir.

Revendications pour l'Etat contractant suivant: ES

**1.** Procédé pour préparer des uréthannes dérivant de fluoro-alcools aliphatiques, d'isocyanates et de composés aromatiques, uréthannes qui répondent à la formule générale 1 :

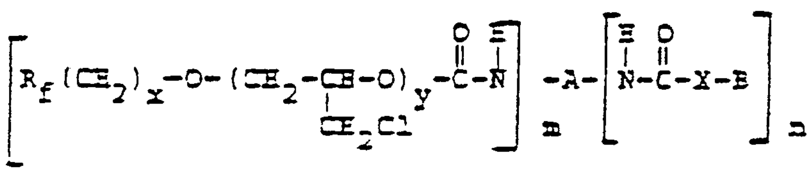

(1)

dans laquelle :

- $R_f$ représente un radical perfluoralkyle contenant de 4 à 20 atomes de carbone, ou un radical $R'_fSO_2NR_1$- dans lequel $R'_f$ a l'une des significations de $R_f$ et $R_1$ représente H ou un alkyle contenant de 1 à 4 atomes de carbone,
- x désigne un nombre entier de 1 à 4,
- y désigne un nombre de 0 à 10,
- m désigne un nombre de 1 à 2,
- n désigne un nombre de 1 à 2, la somme (m + n) étant au plus égale à 3,
- A représente un radical répondant à l'une des formules 2 à 10 suivantes :

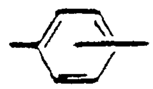 (2) 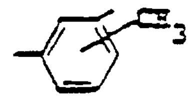 (3)

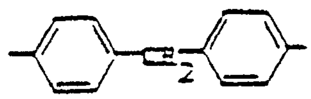 (4) 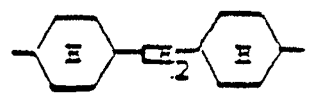 (5)

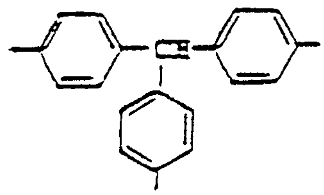 (6) $-(CH_2)_6-$ (7)

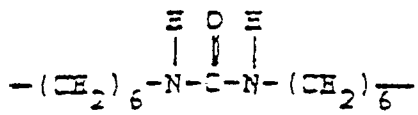 (8)

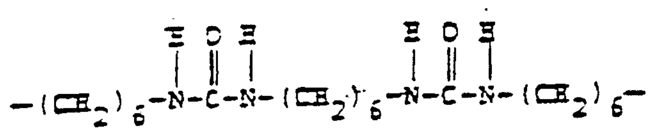 (9)

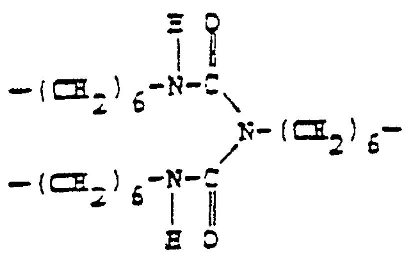 (10)

X représente un radical répondant à l'une des formules 11 à 19 suivantes, qui peut porter un ou plusieurs alkyles en $C_1$-$C_4$ :

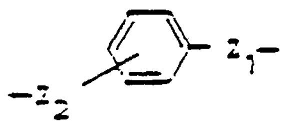 (11) 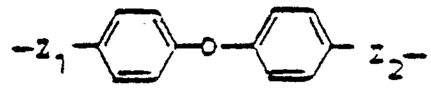 (12)

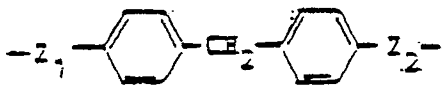 (13) 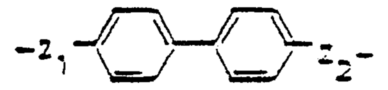 (14)

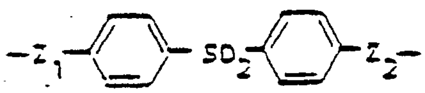 (15) 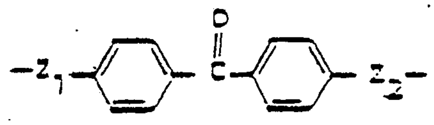 (16)

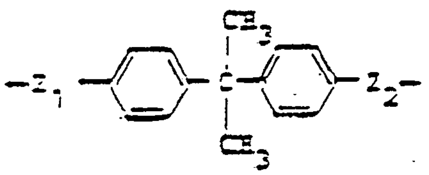 (17) 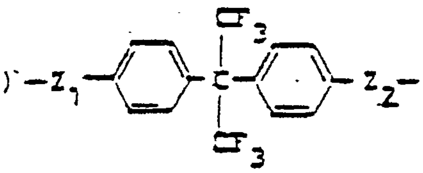 (18)

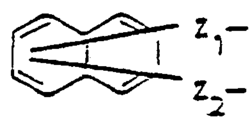 (19) ,

dans lesquelles $Z_1$ et $Z_2$ représentent chacun O, NH ou COO, auquel cas les deux substituants ne sont pas identiques, ou représentent chacun O ou NH, auquel cas les deux substituants peuvent également être identiques,

et

B représente un atome d'hydrogène, un radical alkyle contenant de 1 à 4 atomes de carbone ou un radical répondant à la formule 20 :

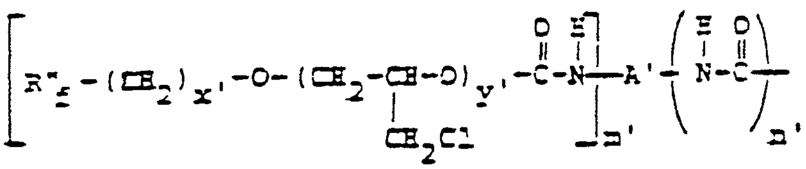 (20) ,

dans laquelle $R''_f$, x', y', m', n' et A' ont chacun une des significations de $R_f$, x, y, m, n et A, procédé caractérisé en ce qu'on fait réagir un fluoro-alcool aliphatique répondant à la formule :

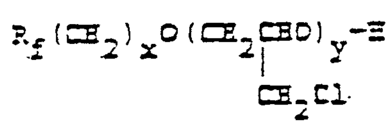 ,

dans laquelle $R_f$, x et y ont les significations indiquées ci-dessus,
avec un di- ou tri-isocyanate correspondant à l'un des radicaux de formules 2 à 10, réaction qui conduit à un produit d'addition répondant à la formule :

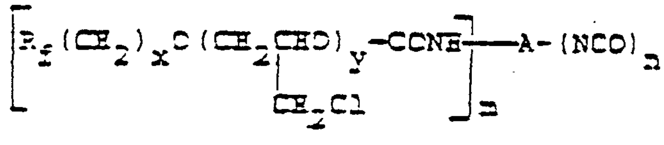

(produit d'addition 1)

dans laquelle $R_f$, x, y, m, n et A ont les significations indiquées ci-dessus,
on fait réagir le produit d'addition 1 avec un composé aromatique dihydroxylique, diaminé, amino-hydroxylique, amino-carboxylique ou hydroxy-carboxylique répondant à l'une des formules suivantes :

H-X-H et H-X-B ,

dans lesquelles X représente un radical répondant à l'une des formules 11 à 19 et B un radical alkyle contenant de 1 à 4 atomes de carbone,
réaction qui conduit aux uréthannes voulues répondant aux formules 21 et 22 :

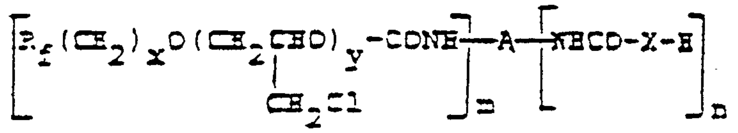

(21)

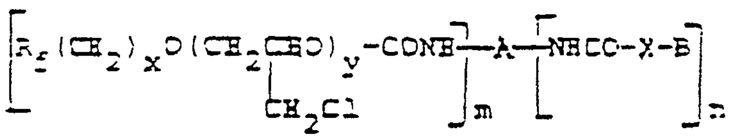

(22),

dans lesquelles $R_f$, x, y, m, n et A ont les significations indiquées ci-dessus et B représente l'alkyle en $C_1$-$C_4$ qui vient d'être mentionné,
et on fait réagir un uréthanne de formule 21 avec un composé contenant un ou plusieurs radicaux isocyanato libres qui correspond au radical de formule 20, de manière à obtenir les uréthannes cherchés qui répondent à la formule 23 :

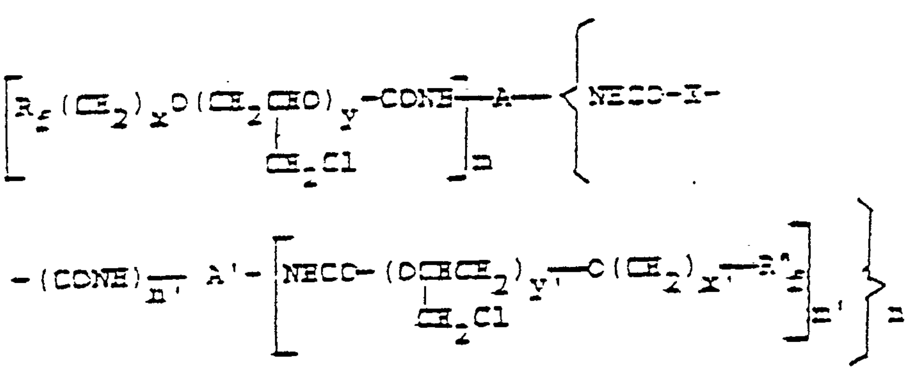

(23),

dans laquelle $R_f$, $R''_f$, x, x', y, y', m, m', n, n', A, A' et X ont les significations qui leur ont été données ci-dessus.

2. Procédé selon la revendication 1 caractérisé en ce qu'on utilise, pour la réaction, des corps de départ tels qu'il en résulte des composés dans lesquels :
   - $R_f$ représente un radical perfluoralkyle contenant de 6 à 16 atomes de carbone,
   - x est égal à 2,
   - y désigne un nombre de 1 à 5,
   - m désigne un nombre de 1 à 2,
   - n désigne un nombre de 1 à 2, la somme (m + n) étant au plus égale à 3,

A représente un radical toluylène, ou un radical répondant à l'une des trois formules 8 à 10,
X représente un radical répondant à l'une des formules 11, 15, 16, 17 et 18, et
B représente H ou un radical de formule 20 dans lequel A' représente un radical répondant à l'une des trois formules 8 à 10.

**3.** Procédé selon la revendication 1 caractérisé en ce qu'on utilise, pour la réaction, des corps de départ tels qu'il en résulte des composés dans lesquels :
$R_f$ représente un radical perfluoralkyle contenant de 6 à 16 atomes de carbone,
x est égal à 2,
y désigne un nombre de 1 à 5,
m désigne un nombre de 1 à 2,
n désigne un nombre de 1 à 2, la somme (m + n) étant au plus égale à 3,
A représente un radical toluylène, ou un radical répondant à l'une des trois formules 8 à 10,
X représente un radical répondant à l'une des formules 11, 15, 16, 17 et 18, et
B représente H ou un radical de formule 20 dans lequel A' représente un radical répondant à l'une des trois formules 8 à 10, $R''_f$ un radical perfluoralkyle contenant de 6 à 16 atomes de carbone, x' le nombre 2, y' un nombre de 1 à 5, m' un nombre de 1 à 2 et n' un nombre de 1 à 2, la somme (m' + n') étant au plus égale à 3.

**4.** Application des uréthannes qui ont été préparés par le procédé des revendications 1 à 3 pour l'ennoblissement oléophobe et hydrophobe de textiles et du cuir.